# EUROPEAN PATENT SPECIFICATION

(11) **EP 1 343 909 B1**
(45) Date of publication and mention of the grant of the patent: **19.08.2009**
(21) Application number: 01996619.1
(22) Date of filing: 08.11.2001
(51) Int. Cl.: C12P 1/04, C12P 21/02, C12N 9/72

(54) **METHODS FOR LARGE SCALE PRODUCTION OF KRINGLE 2 PLUS SERINE PROTEASE DOMAIN (K2S) OF PLASMINOGEN IN PROKARYOTES**
VERFAHREN ZUR PRODUKTION VON PLASMINOGEN-KRINGLE 2 PLUS SERINE PROTEASE DOMÄNE (K2S) IM GROSSMASSSTAB IN PROKARYONTEN
PROCEDES DE PRODUCTION DE DOMAINE KRINGLE 2 PLUS SERINE PROTEASE (K2S) DU PLASMINOGENE A GRANDE ECHELLE DANS DES PROCARYOTES

(30) Priority: 14.11.2000 GB 0027782
(43) Date of publication of application: 17.09.2003
(73) Proprietor: Boehringer Ingelheim International GmbH, 55216 Ingelheim am Rhein (DE)
(72) Inventor: WERNER, Rolf-Günther, 88400 Biberach an der Riss (DE); GOETZ, Friedrich, 72076 Tübingen (DE); TAYAPIWATANA, Chatchai, Janava Rd., BKK (TH); MANOSROI, Jiradej, Nong Hoi, Muang Chiang Mai 50000 (TH); MANOSROI, Aranya, Nong Hoi, Muang Chiang Mai 50000 (TH)
(86) International application number: PCT/EP2001/012920
(87) International publication number: WO 2002/040696

(56) References cited:
- EP-A- 0 357 391
- EP-A- 0 467 676
- EP-A- 1 048 732
- US-A- 6 027 888
- ANDRIS-WIDHOPF J ET AL: "Methods for the generation of chicken monoclonal antibody fragments by phage display" JOURNAL OF IMMUNOLOGICAL METHODS, ELSEVIER SCIENCE PUBLISHERS B.V.,AMSTERDAM, NL, vol. 242, no. 1-2, 28 August 2000 (2000-08-28), pages 159-181, XP004210719 ISSN: 0022-1759
- MANOSROI JIRADEJ ET AL: "Secretion of active recombinant human tissue plasminogen activator derivatives in Escherichia coli." APPLIED AND ENVIRONMENTAL MICROBIOLOGY, vol. 67, no. 6, June 2001 (2001-06), pages 2657-2664, XP002198236 ISSN: 0099-2240
- DATABASE GSN [Online] E.B.I., Hinxton, U.K.; 3 October 2000 (2000-10-03) RADER C AND BARBAS CF III: "Phagemid cloning vector pComb3X" Database accession no. af26281 XP002198237
- HUA Z.-C. ET AL: 'Synthesis and expression of a gene from Kringle-2 domain of tissue plasminogen activator in E. coli.' SCIENCE IN CHINA (SERIES B) vol. 37, no. 6, June 1994, pages 667 - 676
- WALDENSTRÖM M.E. ET AL: 'Synthesis and secretion of a fibrinolytically active tissue-type plasminogen activator in Escherichia coli' GENE vol. 99, 1991, pages 243 - 248
- ROBBENS J. ET AL: 'Improved periplasmic production of biologically active murine interleukin-2 in Escherichia coli through a single amino acid change at the cleavage site' PROCESS BIOCHEMISTRY vol. 41, 2006, pages 1343 - 1346

### Sequence Listing

<110> Boehringer Ingelheim International GmbH
<120> Methods for Large Protein Prpduction in Procaryotes
<130> sequence protocol case 1 1169
<140>
   <141>
<150> GB 0027782.2
   <151> 2000-11-14
<160> 18
<170> PatentIn Ver. 2.1
<210> 1 (SEQ ID NO:1)
   <211> 66
   <212> DNA
   <213> Escherichia coli
<400> 1
   atgaaaaaga cagctatcgc gattgcagtg gcactggctg gtttcgctac cgtggcccag 60
   gcggcc 66
<210> 2 (SEQ ID NO:2)
   <211> 4
   <212> PRT
   <213> Artificial Sequence
<220>
   <223> Description of Artificial Sequence: N-terminal part of K2S molecule
<400> 2
   Ser Glu Gly Asn
   1
<210> 3 (SEQ ID NO:3)
   <211> 6
   <212> PRT
   <213> Artificial Sequence
<220>
   <223> Description of Artificial Sequence: N-terminal part of K2S molecule
<400> 3
   Ser Glu Gly Asn Ser Asp
   1 5
<210> 4 (SEQ ID NO:4)
   <211> 12
   <212> DNA
   <213> Artificial Sequence
<220>
   <223> Description of Artificial Sequence: coding sequence of the N-terminal part of K2S molecule
<400> 4
   tctgagggaa ac 12
<210> 5 (SEQ ID NO:5)
   <211> 18
   <212> DNA
   <213> Artificial Sequence
<220>
   <223> Description of Artificial Sequence: coding sequence of the N-terminal part of K2S molecule
<400> 5
   tctgagggaa acagtgac 18
<210> 6 (SEQ ID NO:6)
   <211> 42
   <212> DNA
   <213> Artificial Sequence
<220>
   <223> Description of Artificial Sequence: oligonucleotid sequence
<400> 6
   gaggaggagg tggcccaggc ggcctctgag ggaaacagtg ac 42
<210> 7 (SEQ ID NO:7)
   <211> 42
   <212> DNA
   <213> Artificial Sequence
<220>
   <223> Description of Artificial Sequence: oligonucleotid sequence
<400> 7
   gaggaggagc tggccggcct ggcccggtcg catgttgtca cg 42
<210> 8 (SEQ ID NO:8)
   <211> 26
   <212> DNA
   <213> Artificial Sequence
<220>
   <223> Description of Artificial Sequence: oligonucleotid sequence
<400> 8
   acatgcgacc gtgacaggcc ggccag 26
<210> 9 (SEQ ID NO:9)
   <211> 26
   <212> DNA
   <213> Artificial Sequence
<220>
   <223> Description of Artificial Sequence: oligonucleotid sequence
<400> 9
   ctggccggcc tgtcacggtc gcatgt 26
<210> 10 (SEQ ID NO:10)
   <211> 354
   <212> PRT
   <213> Artificial Sequence
<220>
   <223> Description of Artificial Sequence: part of the recombinant K2S molecule
<400> 10
   Ser Glu Gly Asn Ser Asp Cys Tyr Phe Gly Asn Gly Ser Ala Tyr Arg
    1               5                  10                  15
   Gly Thr His Ser Leu Thr Glu Ser Gly Ala Ser Cys Leu Pro Trp Asn
               20                  25                  30
   Ser Met Ile Leu Ile Gly Lys Val Tyr Thr Ala Gln Asn Pro Ser Ala
           35                  40                  45
   Gln Ala Leu Gly Leu Gly Lys His Asn Tyr Cys Arg Asn Pro Asp Gly
       50                  55                  60
   Asp Ala Lys Pro Trp Cys His Val Leu Lys Asn Arg Arg Leu Thr Trp
   65                  70                  75                  80
   Glu Tyr Cys Asp Val Pro Ser Cys Ser Thr Cys Gly Leu Arg Gln Tyr
                   85                  90                  95
   Ser Gln Pro Gln Phe Arg Ile Lys Gly Gly Leu Phe Ala Asp Ile Ala
               100                 105                 110
   Ser His Pro Trp Gln Ala Ala Ile Phe Ala Lys His Arg Arg Ser Pro
           115                 120                 125
   Gly Glu Arg Phe Leu Cys Gly Gly Ile Leu Ile Ser Ser Cys Trp Ile
       130                 135                 140
   Leu Ser Ala Ala His Cys Phe Gln Glu Arg Phe Pro Pro His His Leu
   145                 150                 155                 160
   Thr Val Ile Leu Gly Arg Thr Tyr Arg Val Val Pro Gly Glu Glu Glu
                   165                 170                 175
   Gln Lys Phe Glu Val Glu Lys Tyr Ile Val His Lys Glu Phe Asp Asp
               180                 185                 190
   Asp Thr Tyr Asp Asn Asp Ile Ala Leu Leu Gln Leu Lys Ser Asp Ser
           195                 200                 205
   Ser Arg Cys Ala Gln Glu Ser Ser Val Val Arg Thr Val Cys Leu Pro
       210                 215                 220
   Pro Ala Asp Leu Gln Leu Pro Asp Trp Thr Glu Cys Glu Leu Ser Gly
   225                 230                 235                 240
   Tyr Gly Lys His Glu Ala Leu Ser Pro Phe Tyr Ser Glu Arg Leu Lys
                   245                 250                 255
   Glu Ala His Val Arg Leu Tyr Pro Ser Ser Arg Cys Thr Ser Gln His
   260 265 270
   Leu Leu Asn Arg Thr Val Thr Asp Asn Met Leu Cys Ala Gly Asp Thr
   275 280 285
   Arg Ser Gly Gly Pro Gln Ala Asn Leu His Asp Ala Cys Gln Gly Asp
   290 295 300
   Ser Gly Gly Pro Leu Val Cys Leu Asn Asp Gly Arg Met Thr Leu Val
   305 310 315 320
   Gly Ile Ile Ser Trp Gly Leu Gly Cys Gly Gln Lys Asp Val Pro Gly
   325 330 335
   Val Tyr Thr Lys Val Thr Asn Tyr Leu Asp Trp Ile Arg Asp Asn Met
   340 345 350
   Arg Pro
<210> 11 (SEQ ID NO:11)
   <211> 331
   <212> PRT
   <213> Artificial Sequence
<220>
   <223> Description of Artificial Sequence: part of the recombinant K2S molecule
<400> 11
   Ser Gly Ala Ser Cys Leu Pro Trp Asn Ser Met Ile Leu Ile Gly Lys
   1 5 10 15
   Val Tyr Thr Ala Gln Asn Pro Ser Ala Gln Ala Leu Gly Leu Gly Lys
   20 25 30
   His Asn Tyr Cys Arg Asn Pro Asp Gly Asp Ala Lys Pro Trp Cys His
   35 40 45
   Val Leu Lys Asn Arg Arg Leu Thr Trp Glu Tyr Cys Asp Val Pro Ser
   50 55 60
   Cys Ser Thr Cys Gly Leu Arg Gln Tyr Ser Gln Pro Gln Phe Arg Ile
   65 70 75 80
   Lys Gly Gly Leu Phe Ala Asp Ile Ala Ser His Pro Trp Gln Ala Ala
   85 90 95
   Ile Phe Ala Lys His Arg Arg Ser Pro Gly Glu Arg Phe Leu Cys Gly
   100 105 110
   Gly Ile Leu Ile Ser Ser Cys Trp Ile Leu Ser Ala Ala His Cys Phe
   115 120 125
   Gln Glu Arg Phe Pro Pro His His Leu Thr Val Ile Leu Gly Arg Thr
   130 135 140
   Tyr Arg Val Val Pro Gly Glu Glu Glu Gln Lys Phe Glu Val Glu Lys
   145 150 155 160
   Tyr Ile Val His Lys Glu Phe Asp Asp Asp Thr Tyr Asp Asn Asp Ile
                   165                 170                 175
   Ala Leu Leu Gln Leu Lys Ser Asp Ser Ser Arg Cys Ala Gln Glu Ser
               180                 185                 190
   Ser Val Val Arg Thr Val Cys Leu Pro Pro Ala Asp Leu Gln Leu Pro
           195                 200                 205
   Asp Trp Thr Glu Cys Glu Leu Ser Gly Tyr Gly Lys His Glu Ala Leu
       210                 215                 220
   Ser Pro Phe Tyr Ser Glu Arg Leu Lys Glu Ala His Val Arg Leu Tyr
   225                 230                 235                 240
   Pro Ser Ser Arg Cys Thr Ser Gln His Leu Leu Asn Arg Thr Val Thr
                   245                 250                 255
   Asp Asn Met Leu Cys Ala Gly Asp Thr Arg Ser Gly Gly Pro Gln Ala
               260                 265                 270
   Asn Leu His Asp Ala Cys Gln Gly Asp Ser Gly Gly Pro Leu Val Cys
           275                 280                 285
   Leu Asn Asp Gly Arg Met Thr Leu Val Gly Ile Ile Ser Trp Gly Leu
       290                 295                 300
   Gly Cys Gly Gln Lys Asp Val Pro Gly Val Tyr Thr Lys Val Thr Asn
   305                 310                 315                 320
   Tyr Leu Asp Trp Ile Arg Asp Asn Met Arg Pro
                   325                 330
<210> 12 (SEQ ID NO:12)
   <211> 339
   <212> PRT
   <213> Artificial Sequence
<220>
   <223> Description of Artificial Sequence: part of the recombinant K2S molecule (modified)
<400> 12
   Ser Glu Gly Asn Ser Leu Thr Glu Ser Gly Ala Ser Cys Leu Pro Trp
    1               5                  10                  15
   Asn Ser Met Ile Leu Ile Gly Lys Val Tyr Thr Ala Gln Asn Pro Ser
               20                  25                  30
   Ala Gln Ala Leu Gly Leu Gly Lys His Asn Tyr Cys Arg Asn Pro Asp
           35                  40                  45
   Gly Asp Ala Lys Pro Trp Cys His Val Leu Lys Asn Arg Arg Leu Thr
       50                  55                  60
   Trp Glu Tyr Cys Asp Val Pro Ser Cys Ser Thr Cys Gly Leu Arg Gln
   65                  70                  75                  80
   Tyr Ser Gln Pro Gln Phe Arg Ile Lys Gly Gly Leu Phe Ala Asp Ile
                   85                  90                  95
   Ala Ser His Pro Trp Gln Ala Ala Ile Phe Ala Lys His Arg Arg Ser
               100                 105                 110
   Pro Gly Glu Arg Phe Leu Cys Gly Gly Ile Leu Ile Ser Ser Cys Trp
           115                 120                 125
   Ile Leu Ser Ala Ala His Cys Phe Gln Glu Arg Phe Pro Pro His His
       130                 135                 140
   Leu Thr Val Ile Leu Gly Arg Thr Tyr Arg Val Val Pro Gly Glu Glu
   145                 150                 155                 160
   Glu Gln Lys Phe Glu Val Glu Lys Tyr Ile Val His Lys Glu Phe Asp
                   165                 170                 175
   Asp Asp Thr Tyr Asp Asn Asp Ile Ala Leu Leu Gln Leu Lys Ser Asp
               180                 185                 190
   Ser Ser Arg Cys Ala Gln Glu Ser Ser Val Val Arg Thr Val Cys Leu
           195                 200                 205
   Pro Pro Ala Asp Leu Gln Leu Pro Asp Trp Thr Glu Cys Glu Leu Ser
       210                 215                 220
   Gly Tyr Gly Lys His Glu Ala Leu Ser Pro Phe Tyr Ser Glu Arg Leu
   225                 230                 235                 240
   Lys Glu Ala His Val Arg Leu Tyr Pro Ser Ser Arg Cys Thr Ser Gln
                   245                 250                 255
   His Leu Leu Asn Arg Thr Val Thr Asp Asn Met Leu Cys Ala Gly Asp
               260                 265                 270
   Thr Arg Ser Gly Gly Pro Gln Ala Asn Leu His Asp Ala Cys Gln Gly
           275                 280                 285
   Asp Ser Gly Gly Pro Leu Val Cys Leu Asn Asp Gly Arg Met Thr Leu
       290                 295                 300
   Val Gly Ile Ile Ser Trp Gly Leu Gly Cys Gly Gln Lys Asp Val Pro
   305                 310                 315                 320
   Gly Val Tyr Thr Lys Val Thr Asn Tyr Leu Asp Trp Ile Arg Asp Asn
                   325                 330                 335
   Met Arg Pro
<210> 13 (SEQ ID NO:13)
   <211> 335
   <212> PRT
   <213> Artificial Sequence
<220>
   <223> Description of Artificial Sequence: part of the recombinant K2S molecule (modified)
<400> 13
   Ser Leu Thr Glu Ser Gly Ala Ser Cys Leu Pro Trp Asn Ser Met Ile
   1 5 10 15
   Leu Ile Gly Lys Val Tyr Thr Ala Gln Asn Pro Ser Ala Gln Ala Leu
   20 25 30
   Gly Leu Gly Lys His Asn Tyr Cys Arg Asn Pro Asp Gly Asp Ala Lys
   35 40 45
   Pro Trp Cys His Val Leu Lys Asn Arg Arg Leu Thr Trp Glu Tyr Cys
   50 55 60
   Asp Val Pro Ser Ser Ser Thr Cys Gly Leu Arg Gln Tyr Ser Gln Pro
   65 70 75 80
   Gln Phe Arg Ile Lys Gly Gly Leu Phe Ala Asp Ile Ala Ser His Pro
   85 90 95
   Trp Gln Ala Ala Ile Phe Ala Lys His Arg Arg Ser Pro Gly Glu Arg
   100 105 110
   Phe Leu Cys Gly Gly Ile Leu Ile Ser Ser Cys Trp Ile Leu Ser Ala
   115 120 125
   Ala His Cys Phe Gln Glu Arg Phe Pro Pro His His Leu Thr Val Ile
   130 135 140
   Leu Gly Arg Thr Tyr Arg Val Val Pro Gly Glu Glu Glu Gln Lys Phe
   145 150 155 160
   Glu Val Glu Lys Tyr Ile Val His Lys Glu Phe Asp Asp Asp Thr Tyr
   165 170 175
   Asp Asn Asp Ile Ala Leu Leu Gln Leu Lys Ser Asp Ser Ser Arg Cys
   180 185 190
   Ala Gln Glu Ser Ser Val Val Arg Thr Val Cys Leu Pro Pro Ala Asp
   195 200 205
   Leu Gln Leu Pro Asp Trp Thr Glu Cys Glu Leu Ser Gly Tyr Gly Lys
   210 215 220
   His Glu Ala Leu Ser Pro Phe Tyr Ser Glu Arg Leu Lys Glu Ala His
   225 230 235 240
   Val Arg Leu Tyr Pro Ser Ser Arg Cys Thr Ser Gln His Leu Leu Asn
   245 250 255
   Arg Thr Val Thr Asp Asn Met Leu Cys Ala Gly Asp Thr Arg Ser Gly
   260 265 270
   Gly Pro Gln Ala Asn Leu His Asp Ala Cys Gln Gly Asp Ser Gly Gly
   275 280 285
   Pro Leu Val Cys Leu Asn Asp Gly Arg Met Thr Leu Val Gly Ile Ile
   290 295 300
   Ser Trp Gly Leu Gly Cys Gly Gln Lys Asp Val Pro Gly Val Tyr Thr
   305 310 315 320
   Lys Val Thr Asn Tyr Leu Asp Trp Ile Arg Asp Asn Met Arg Pro
   325 330 335
<210> 14 (SEQ ID NO:14)
   <211> 343
   <212> PRT
   <213> Artificial Sequence
<220>
   <223> Description of Artificial Sequence: part of the recombinant K2S molecule (modified)
<400> 14
   Ser Glu Gly Asn Ser Asp Thr His Ser Leu Thr Glu Ser Gly Ala Ser
    1               5                   10                  15
   Cys Leu Pro Trp Asn Ser Met Ile Leu Ile Gly Lys Val Tyr Thr Ala
               20                  25                  30
   Gln Asn Pro Ser Ala Gln Ala Leu Gly Leu Gly Lys His Asn Tyr Cys
           35                  40                  45
   Arg Asn Pro Asp Gly Asp Ala Lys Pro Trp Cys His Val Leu Lys Asn
       50                  55                  60
   Arg Arg Leu Thr Trp Glu Tyr Cys Asp Val Pro Ser Cys Ser Thr Cys
   65                  70                  75                  80
   Gly Leu Arg Gln Tyr Ser Gln Pro Gln Phe Arg Ile Lys Gly Gly Leu
                   85                  90                  95
   Phe Ala Asp Ile Ala Ser His Pro Trp Gln Ala Ala Ile Phe Ala Lys
               100                 105                 110
   His Arg Arg Ser Pro Gly Glu Arg Phe Leu Cys Gly Gly Ile Leu Ile
           115                 120                 125
   Ser Ser Cys Trp Ile Leu Ser Ala Ala His Cys Phe Gln Glu Arg Phe
       130                 135                 140
   Pro Pro His His Leu Thr Val Ile Leu Gly Arg Thr Tyr Arg Val Val
   145                 150                 155                 160
   Pro Gly Glu Glu Glu Gln Lys Phe Glu Val Glu Lys Tyr Ile Val His
                   165                 170                 175
   Lys Glu Phe Asp Asp Asp Thr Tyr Asp Asn Asp Ile Ala Leu Leu Gln
               180                 185                 190
   Leu Lys Ser Asp Ser Ser Arg Cys Ala Gln Glu Ser Ser Val Val Arg
           195                 200                 205
   Thr Val Cys Leu Pro Pro Ala Asp Leu Gln Leu Pro Asp Trp Thr Glu
       210                 215                 220
   Cys Glu Leu Ser Gly Tyr Gly Lys His Glu Ala Leu Ser Pro Phe Tyr
   225                 230                 235                 240
   Ser Glu Arg Leu Lys Glu Ala His Val Arg Leu Tyr Pro Ser Ser Arg
                   245                 250                 255
   Cys Thr Ser Gln His Leu Leu Asn Arg Thr Val Thr Asp Asn Met Leu
               260                 265                 270
   Cys Ala Gly Asp Thr Arg Ser Gly Gly Pro Gln Ala Asn Leu His Asp
           275                 280                 285
   Ala Cys Gln Gly Asp Ser Gly Gly Pro Leu Val Cys Leu Asn Asp Gly
       290                 295                 300
   Arg Met Thr Leu Val Gly Ile Ile Ser Trp Gly Leu Gly Cys Gly Gln
   305                 310                 315                 320
   Lys Asp Val Pro Gly Val Tyr Thr Lys Val Thr Asn Tyr Leu Asp Trp
                   325                 330                 335
   Ile Arg Asp Asn Met Arg Pro
   340
<210> 15 (SEQ ID NO:15)
   <211> 343
   <212> PRT
   <213> Artificial Sequence
<220>
   <223> Description of Artificial Sequence: part of the recombinant K2S molecule (modified)
<400> 15
   Ser Glu Gly Asn Ser Asp Thr His Ser Leu Thr Glu Ser Gly Ala Ser
    1               5                  10                  15
   Cys Leu Pro Trp Asn Ser Met Ile Leu Ile Gly Lys Val Tyr Thr Ala
               20                  25                  30
   Gln Asn Pro Ser Ala Gln Ala Leu Gly Leu Gly Lys His Asn Tyr Cys
           35                  40                  45
   Arg Asn Pro Asp Gly Asp Ala Lys Pro Trp Cys His Val Leu Lys Asn
       50                  55                  60
   Arg Arg Leu Thr Trp Glu Tyr Cys Asp Val Pro Ser Ser Ser Thr Cys
   65                  70                  75                  80
   Gly Leu Arg Gln Tyr Ser Gln Pro Gln Phe Arg Ile Lys Gly Gly Leu
                   85                  90                  95
   Phe Ala Asp Ile Ala Ser His Pro Trp Gln Ala Ala Ile Phe Ala Lys
               100                 105                 110
   His Arg Arg Ser Pro Gly Glu Arg Phe Leu Cys Gly Gly Ile Leu Ile
           115                 120                 125
   Ser Ser Cys Trp Ile Leu Ser Ala Ala His Cys Phe Gln Glu Arg Phe
       130                 135                 140
   Pro Pro His His Leu Thr Val Ile Leu Gly Arg Thr Tyr Arg Val Val
   145                 150                 155                 160
   Pro Gly Glu Glu Glu Gln Lys Phe Glu Val Glu Lys Tyr Ile Val His
                   165                 170                 175
   Lys Glu Phe Asp Asp Asp Thr Tyr Asp Asn Asp Ile Ala Leu Leu Gln
               180                 185                 190
   Leu Lys Ser Asp Ser Ser Arg Cys Ala Gln Glu Ser Ser Val Val Arg
           195                 200                 205
   Thr Val Cys Leu Pro Pro Ala Asp Leu Gln Leu Pro Asp Trp Thr Glu
       210                 215                 220
   Cys Glu Leu Ser Gly Tyr Gly Lys His Glu Ala Leu Ser Pro Phe Tyr
   225                 230                 235                 240
   Ser Glu Arg Leu Lys Glu Ala His Val Arg Leu Tyr Pro Ser Ser Arg
                   245                 250                 255
   Cys Thr Ser Gln His Leu Leu Asn Arg Thr Val Thr Asp Asn Met Leu
                   260                 265                 270
   Cys Ala Gly Asp Thr Arg Ser Gly Gly Pro Gln Ala Asn Leu His Asp
           275                 280                 285
   Ala Cys Gln Gly Asp Ser Gly Gly Pro Leu Val Cys Leu Asn Asp Gly
       290                 295                 300
   Arg Met Thr Leu Val Gly Ile Ile Ser Trp Gly Leu Gly Cys Gly Gln
   305                 310                 315                 320
   Lys Asp Val Pro Gly Val Tyr Thr Lys Val Thr Asn Tyr Leu Asp Trp
                   325                 330                 335
   Ile Arg Asp Asn Met Arg Pro
               340
<210> 16 (SEQ ID NO:16)
   <211> 308
   <212> PRT
   <213> Artificial Sequence
<220>
   <223> Description of Artificial Sequence: part of the recombinant K2S molecule (modified)
<400> 16
   Ser Ala Gln Ala Leu Gly Leu Gly Lys His Asn Tyr Cys Arg Asn Pro
    1               5                  10                  15
   Asp Gly Asp Ala Lys Pro Trp Cys His Val Leu Lys Asn Arg Arg Leu
               20                  25                  30
   Thr Trp Glu Tyr Cys Asp Val Pro Ser Cys Ser Thr Cys Gly Leu Arg
           35                  40                  45
   Gln Tyr Ser Gln Pro Gln Phe Arg Ile Lys Gly Gly Leu Phe Ala Asp
       50                  55                  60
   Ile Ala Ser His Pro Trp Gln Ala Ala Ile Phe Ala Lys His Arg Arg
   65                  70                  75                  80
   Ser Pro Gly Glu Arg Phe Leu Cys Gly Gly Ile Leu Ile Ser Ser Cys
                   85                  90                  95
   Trp Ile Leu Ser Ala Ala His Cys Phe Gln Glu Arg Phe Pro Pro His
               100                 105                 110
   His Leu Thr Val Ile Leu Gly Arg Thr Tyr Arg Val Val Pro Gly Glu
           115                 120                 125
   Glu Glu Gln Lys Phe Glu Val Glu Lys Tyr Ile Val His Lys Glu Phe
       130                 135                 140
   Asp Asp Asp Thr Tyr Asp Asn Asp Ile Ala Leu Leu Gln Leu Lys Ser
   145                 150                 155                 160
   Asp Ser Ser Arg Cys Ala Gln Glu Ser Ser Val Val Arg Thr Val Cys
                   165                 170                 175
   Leu Pro Pro Ala Asp Leu Gln Leu Pro Asp Trp Thr Glu Cys Glu Leu
               180                 185                 190
   Ser Gly Tyr Gly Lys His Glu Ala Leu Ser Pro Phe Tyr Ser Glu Arg
           195                 200                 205
   Leu Lys Glu Ala His Val Arg Leu Tyr Pro Ser Ser Arg Cys Thr Ser
       210                 215                 220
   Gln His Leu Leu Asn Arg Thr Val Thr Asp Asn Met Leu Cys Ala Gly
   225                 230                 235                 240
   Asp Thr Arg Ser Gly Gly Pro Gln Ala Asn Leu His Asp Ala Cys Gln
                   245                 250                 255
   Gly Asp Ser Gly Gly Pro Leu Val Cys Leu Asn Asp Gly Arg Met Thr
               260                 265                 270
   Leu Val Gly Ile Ile Ser Trp Gly Leu Gly Cys Gly Gln Lys Asp Val
           275                 280                 285
   Pro Gly Val Tyr Thr Lys Val Thr Asn Tyr Leu Asp Trp Ile Arg Asp
       290                 295                 300
   Asn Met Arg Pro
   305
<210> 17 (SEQ ID NO:17)
   <211> 268
   <212> PRT
   <213> Artificial Sequence
<220>
   <223> Description of Artificial Sequence: part of the recombinant K2S molecule (modified)
<400> 17
   Ser Cys Ser Thr Cys Gly Leu Arg Gln Tyr Ser Gln Pro Gln Phe Arg
    1               5                  10                  15
   Ile Lys Gly Gly Leu Phe Ala Asp Ile Ala Ser His Pro Trp Gln Ala
               20                  25                  30
   Ala Ile Phe Ala Lys His Arg Arg Ser Pro Gly Glu Arg Phe Leu Cys
           35                  40                  45
   Gly Gly Ile Leu Ile Ser Ser Cys Trp Ile Leu Ser Ala Ala His Cys
       50                  55                  60
   Phe Gln Glu Arg Phe Pro Pro His His Leu Thr Val Ile Leu Gly Arg
   65                  70                  75                  80
   Thr Tyr Arg Val Val Pro Gly Glu Glu Glu Gln Lys Phe Glu Val Glu
                   85                  90                  95
   Lys Tyr Ile Val His Lys Glu Phe Asp Asp Asp Thr Tyr Asp Asn Asp
               100                 105                 110
   Ile Ala Leu Leu Gln Leu Lys Ser Asp Ser Ser Arg Cys Ala Gln Glu
           115                 120                 125
   Ser Ser Val Val Arg Thr Val Cys Leu Pro Pro Ala Asp Leu Gln Leu
       130                 135                 140
   Pro Asp Trp Thr Glu Cys Glu Leu Ser Gly Tyr Gly Lys His Glu Ala
   145                 150                 155                 160
   Leu Ser Pro Phe Tyr Ser Glu Arg Leu Lys Glu Ala His Val Arg Leu
                   165                 170                 175
   Tyr Pro Ser Ser Arg Cys Thr Ser Gln His Leu Leu Asn Arg Thr Val
               180                 185                 190
   Thr Asp Asn Met Leu Cys Ala Gly Asp Thr Arg Ser Gly Gly Pro Gln
           195                 200                 205
   Ala Asn Leu His Asp Ala Cys Gln Gly Asp Ser Gly Gly Pro Leu Val
       210                 215                 220
   Cys Leu Asn Asp Gly Arg Met Thr Leu Val Gly Ile Ile Ser Trp Gly
   225                 230                 235                 240
   Leu Gly Cys Gly Gln Lys Asp Val Pro Gly Val Tyr Thr Lys Val Thr
                   245                 250                 255
   Asn Tyr Leu Asp Trp Ile Arg Asp Asn Met Arg Pro
               260                 265
<210> 18 (SEQ ID NO:18)
   <211> 527
   <212> PRT
   <213> Homo sapiens (tPA)
<400> 18
   Ser Tyr Gln Val Ile Cys Arg Asp Glu Lys Thr Gln Met Ile Tyr Gln
    1               5                  10                  15
   Gln His Gln Ser Trp Leu Arg Pro Val Leu Arg Ser Asn Arg Val Glu
               20                  25                  30
   Tyr Cys Trp Cys Asn Ser Gly Arg Ala Gln Cys His Ser Val Pro Val
           35                  40                  45
   Lys Ser Cys Ser Glu Pro Arg Cys Phe Asn Gly Gly Thr Cys Gln Gln
       50                  55                  60
   Ala Leu Tyr Phe Ser Asp Phe Val Cys Gln Cys Pro Glu Gly Phe Ala
   65                  70                  75                  80
   Gly Lys Cys Cys Glu Ile Asp Thr Arg Ala Thr Cys Tyr Glu Asp Gln
                   85                  90                  95
   Gly Ile Ser Tyr Arg Gly Thr Trp Ser Thr Ala Glu Ser Gly Ala Glu
               100                 105                 110
   Cys Thr Asn Trp Asn Ser Ser Ala Leu Ala Gln Lys Pro Tyr Ser Gly
           115                 120                 125
   Arg Arg Pro Asp Ala Ile Arg Leu Gly Leu Gly Asn His Asn Tyr Cys
       130                 135                 140
   Arg Asn Pro Asp Arg Asp Ser Lys Pro Trp Cys Tyr Val Phe Lys Ala
   145                 150                 155                 160
   Gly Lys Tyr Ser Ser Glu Phe Cys Ser Thr Pro Ala Cys Ser Glu Gly
                   165                 170                 175
   Asn Ser Asp Cys Tyr Phe Gly Asn Gly Ser Ala Tyr Arg Gly Thr His
               180                 185                 190
   Ser Leu Thr Glu Ser Gly Ala Ser Cys Leu Pro Trp Asn Ser Met Ile
           195                 200                 205
   Leu Ile Gly Lys Val Tyr Thr Ala Gln Asn Pro Ser Ala Gln Ala Leu
       210                 215                 220
   Gly Leu Gly Lys His Asn Tyr Cys Arg Asn Pro Asp Gly Asp Ala Lys
   225                 230                 235                 240
   Pro Trp Cys His Val Leu Lys Asn Arg Arg Leu Thr Trp Glu Tyr Cys
                   245                 250                 255
   Asp Val Pro Ser Cys Ser Thr Cys Gly Leu Arg Gln Tyr Ser Gln Pro
               260                 265                 270
   Gln Phe Arg Ile Lys Gly Gly Leu Phe Ala Asp Ile Ala Ser His Pro
           275                 280                 285
   Trp Gln Ala Ala Ile Phe Ala Lys His Arg Arg Ser Pro Gly Glu Arg
       290                 295                 300
   Phe Leu Cys Gly Gly Ile Leu Ile Ser Ser Cys Trp Ile Leu Ser Ala
   305                 310                 315                 320
   Ala His Cys Phe Gln Glu Arg Phe Pro Pro His His Leu Thr Val Ile
                   325                 330                 335
   Leu Gly Arg Thr Tyr Arg Val Val Pro Gly Glu Glu Glu Gln Lys Phe
               340                 345                 350
   Glu Val Glu Lys Tyr Ile Val His Lys Glu Phe Asp Asp Asp Thr Tyr
           355                 360                 365
   Asp Asn Asp Ile Ala Leu Leu Gln Leu Lys Ser Asp Ser Ser Arg Cys
       370                 375                 380
   Ala Gln Glu Ser Ser Val Val Arg Thr Val Cys Leu Pro Pro Ala Asp
   385                 390                 395                 400
   Leu Gln Leu Pro Asp Trp Thr Glu Cys Glu Leu Ser Gly Tyr Gly Lys
                   405                 410                 415
   His Glu Ala Leu Ser Pro Phe Tyr Ser Glu Arg Leu Lys Glu Ala His
               420                 425                 430
   Val Arg Leu Tyr Pro Ser Ser Arg Cys Thr Ser Gln His Leu Leu Asn
           435                 440                 445
   Arg Thr Val Thr Asp Asn Met Leu Cys Ala Gly Asp Thr Arg Ser Gly
       450                 455                 460
   Gly Pro Gln Ala Asn Leu His Asp Ala Cys Gln Gly Asp Ser Gly Gly
   465                 470                 475                 480
   Pro Leu Val Cys Leu Asn Asp Gly Arg Met Thr Leu Val Gly Ile Ile
                   485                 490                 495
   Ser Trp Gly Leu Gly Cys Gly Gln Lys Asp Val Pro Gly Val Tyr Thr
               500                 505                 510
   Lys Val Thr Asn Tyr Leu Asp Trp Ile Arg Asp Asn Met Arg Pro
           515                 520                 525

## Description

The invention belongs to the field of protein production in prokaryotic cells.

The invention relates to methods for the production of recombinant DNA-derived kringle 2 plus serine protease (K2S) domain of human tissue plasminogen activator (EPA) in prokaryotic cells, wherein said K2S is secreted extracellularly as an active and correctly folded protein, and the prokaryotic cell contains and expresses a vector comprising the DNA coding for said heterologous protein operably linked to the DNA coding for the signal peptide OmpA.

### Background art

Prokaryotic expression systems for heterologous proteins are commonly used for proteins which do not require mammalian glycosylation patterns as they provide a cheap way of producing large quantities of said protein. The formation of highly aggregated protein or inclusion bodies can be commonly found in high-level expression of many heterologous proteins in *E.coli*. One way of protein production is via inclusion bodies which develop in cytoplasm. Cell wall and outer membrane components of the prokaryotic cells used for production (e.g. *E.coli*) usually contaminate the cell lysate containing the heterologous protein when said inclusion bodies are prepared by low-speed centrifugation. The outer membrane component can be eliminated by selective extraction with detergents and low concentrations of either urea or guanidine-HCl.

One example of such a heterologous protein is a tPA derivative.

Tissue plasminogen activator (tPA) is a polypeptide containing 527 amino acid residues (27) with a molecular mass of 72 kDa. The molecule is divided into five structural domains. Nearby the N-terminal region is a looped finger domain, which is followed by a growth factor domain. Two similar domains, kringle 1 and kringle 2, are following. Both finger and kringle 2 domains bind specifically to the fibrin clots thereby accelerating tPA protein activation of bound plasminogen. Downstream of kringle 2 is the serine protease, with its catalytic site located at the C-terminus. The serine protease is responsible for converting plasminogen to plasmin a reaction important in the homeostasis of fibrin formation and clot dissolution. The correct folding of tPA requires the correct pairing of 17 disulfide bridges in the molecule (1).

Clinically, tPA is a thrombolytic agent of choice for the treatment of acute myocardial infarction. It has the advantage of causing no side effects on systemic haemorrhaging and fibrinogen depletion (7). Bowes melanoma cells were first used as a source in tPA production for therapeutic purposes (12). Since a consistent process with efficient production of highly purified protein in good yield is required for clinical use, the construction of full-length recombinant-tPA (r-tPA) progressed to mammalian cells. Chinese hamster ovary cells were transfected with the tPA gene to synthesize the r-tPA (8, 22). The recombinant product produced by a mammalian fermentation system was harvested from the culture medium. Attracted by simplicity and economy of production, a number of efforts in producing r-tPA from bacteria, especially from *Escherichia coli,* were investigated (10, 13, 30). Regarding the low yield and the formation of inclusion bodies, which resulted in misfolding and in an inactive enzyme, numerous strategies have been proposed to overcome these problems. The major criterion is to synthesize the smallest molecule, which is still active instead of full-length tPA.

Several deletion-mutant variants including kringle 2 plus serine protease (*K2S*) were considered. However, the enzymatic activity of the recombinant-K2S (r-K2S) was obtained only when refolding processes of purified inclusion bodies from cytoplasmic compartment were achieved (16, 29). In order to avoid the cumbersome refolding processes and periplasmic protein delivery, special bacterial expression systems were exploited (6, 31). Despite periplasmic expression of tPA, overexpression led to inactive aggregates, even in the relatively high oxidizing condition in the periplasm.

In the prior art, there are a few descriptions of methods for the preparation of recombinant K2S in E. coli. However, there is no disclosure of a method leading to a cost effective method for large scale production of biologically active K2S.

Obukowicz et al. (25) expressed and purified r-K2S from periplasmic space. The obvious disadvantage of this method was an extra periplasmic extraction step, which is not suitable for large scale production.

Saito et al. (29) disclose the cytoplasmic expression of r-K2S. The authors used an in vivo renaturation processes for the expressed r-K2S, which was purified from the cytoplasmic space of E. coli as inclusion body. Boehringer Mannheim use a similar cumbersome denaturing/refolding process involving the steps of cell digestion, solubilization under denaturing and reducing conditions and reactivation under oxidizing conditions in the presence of GSH/GSSG which is not cost effective and requires mutation of the amino acid sequence (24).

In 1991, Waldenström et al. (34) constructed a vector (pEZZK2P) for the secretion of kringle 2 plus serine protease domain to E. coli culture supernatant. Hydroxylamine was used to remove the ZZ fusion peptide from IgG-Sepharose purified fraction. The cleavage agent hydroxylamine required modification of the cleavage sites of kringle 2 plus serine protease (Asn¹⁷⁷ → Ser and Asn¹⁸⁴ → Gln) thus to protect it from hydroxylamine digestion. However, the resulting non-native, not properly folded K2S molecule is not suitable for therapeutic purposes. The unusual sequence may even activate the human immune system.

Hua et al. (37) show secretion of an OmpA- K₂ fusion polypeptide into the periplasm of E. coli.

The problem underlying the present invention was thus to provide a commercially applicable method for large scale production of K2S, wherein said protein is secreted in its biologically active form into the culture supernatant.

### Description of the invention

The problem was solved within the scope of the claims and specification of the present invention.

The use of the singular or plural in the claims or specification is in no way intended to be limiting and also includes the other form.

The invention relates to a method for the production of recombinant DNA-derived K2S in prokaryotic cells, wherein said heterologous protein is secreted extracellularly as an active and correctly folded protein, characterized in that the prokaryotic cell contains and expresses a vector comprising the DNA coding for said heterologous protein operably linked to the DNA coding for the signal peptide OmpA.

Surprisingly, the use of the signal peptide OmpA in combination with the N-terminal amino acids SEGNSD (SEQ ID NO:3) translocate the recombinant DNA-derived proteins to the outer surface and facilitates the release of the functional and active molecule into the culture medium to a greater extent than any other method in the prior art. Before crossing the outer membrane, the recombinant DNA-derived protein is correctly folded according to the method of the present invention. The signal peptide is cleaved off to produce a mature molecule. Surprisingly, the efficiency of signal peptide removal is very high and leads to correct folding of the recombinant DNA-derived protein. This method exemplified for the kringle 2 plus serine protease domain (K2S) of tissue plasminogen activator protein in example 1, is also described for expression of several different proteins and polypeptides which do not require mammalian glycosylation in prokaryotic host cells.

The method according to the invention has advantages over methods known in the art- not only that it is a cheap production method due to the prokaryotic host cell used, surprisingly, a correctly folded molecule is secreted to the supernatant.

The skilled person can easily obtain the DNA sequence of K2S to be expressed by the method according to the invention from suitable databases and clone it to be used in the method according to the invention.

Said signal peptide OmpA interacts with SecE and is delivered across the inner membrane by energy generated by SecA, which binds to Sec components (SecE-SecY). SecY forms a secretion pore to dispatch the recombinant DNA-derived protein according to the invention. The space between the outer membrane and inner membrane of Gram-negative bacteria, periplasm, has higher oxidative condition in comparison to the cytoplasmic space. This supports the formation of disulfide bonds and properly folding of K2S in the periplasm to yield an active molecule. According to the present invention, the signal peptide will be cleaved off to produce a mature molecule. The complex of GspD secretin and GspS lipoprotein on the outer membrane serves as gate channel for secreting the recombinant protein according to the invention to the extracellular medium. This secretion process requires energy, which is generated in cytoplasm by GspE nucleotide-binding protein then transferred to the inner membrane protein (Gsp G-J, F and K-N). GspC transfers the energy to GspD by forming a cross-linker between a set of inner membrane protein (Gsp G-J, F and K-N) and GspD. Before crossing the outer membrane successfully, the recombinant protein is correctly folded.

Operably linked according to the invention means that the DNA encoding K2S comprising the nucleic acid encoding SEGNSD at its N-terminal portion is cloned directly downstream of the OmpA DNA into the vector in order to achieve expression of the OmpA-heterologous protein-fusion protein and to direct secretion outside the prokaryotic host cell. Typically, the majority of K2S is secreted and can then be purified by appropriate methods such as ammonium sulfate precipitation. The invention also includes the use of inducers such as IPTG or IPTG in combination with glycerol, the improvement of the incubation condition and harvesting period to maximize the amount of active protein.

The inventors surprisingly found that the OmpA signal peptide operatively linked to the amino acids characterized by the sequence SEGNSD (SEQ ID NO:3) lead to secretion of the heterologous protein into the medium rather than accumulation in the periplasmatic space.

Said DNA encoding the OmpA signal peptide is fused to a short peptide characterized by the amino acid sequence SEGNSD (SEQ ID NO:3) or the coding nucleic acid sequence TCTGAGGGAAACAGTGAC (SEQ ID NO:5) and located in the N-terminal portion of K2S. Thus, said fusion protein consisits of OmpA-SEGNSD K2S.

Thus, in a preferred method according to the invention said the prokaryotic cell contains and expresses a vector comprising the DNA coding for K2S operably linked to the DNA coding for the signal peptide OmpA which is operably linked to the nucleic acid molecule defined by the sequence TCTGAGGGAAACAGTGAC (SEQ ID NO:5).

The method according to the invention comprises prokaryotic host cells such as, but not limited to *Escherichia coli (E. coli), Bacillus subtilis, Streptomyces, Pseudomonas, e.g. Pseudomonas putida, Proteus mirabilis* or *Staphylococcus,* e.g. *Staphylococcus carnosus*. Preferably said host cells according to the invention are Gram-negative bacteria.

Preferably, a method according to the invention is also characterised in that the prokaryotic cell is *E. coli.* Suitable strains include, but are not limited to *E.coli* XL-1 blue, *E.coli* BL21(DE3), *E.coli* JM109, *E.coli* DH series, *E.coli* TOP10 and *E.coli* HB101.

Preferably, a method according to the invention is also characterised in that the following steps are carried out:
a) the DNA encoding K2S is amplified by PCR;
b) the PCR product is purified;
c) said PCR product is inserted into a vector comprising the DNA coding for OmpA signal peptide and the DNA coding for gpIII in such a way that said PCR product is operably linked upstream to the DNA coding for the OmpA signal sequence and linked downstream to the DNA coding for gpIII of said vector;
d) that a stop codon is inserted between said heterologous protein and gpIII;
e) said vector is expressed by the prokaryotic cell
f) the heterologous protein is purified.

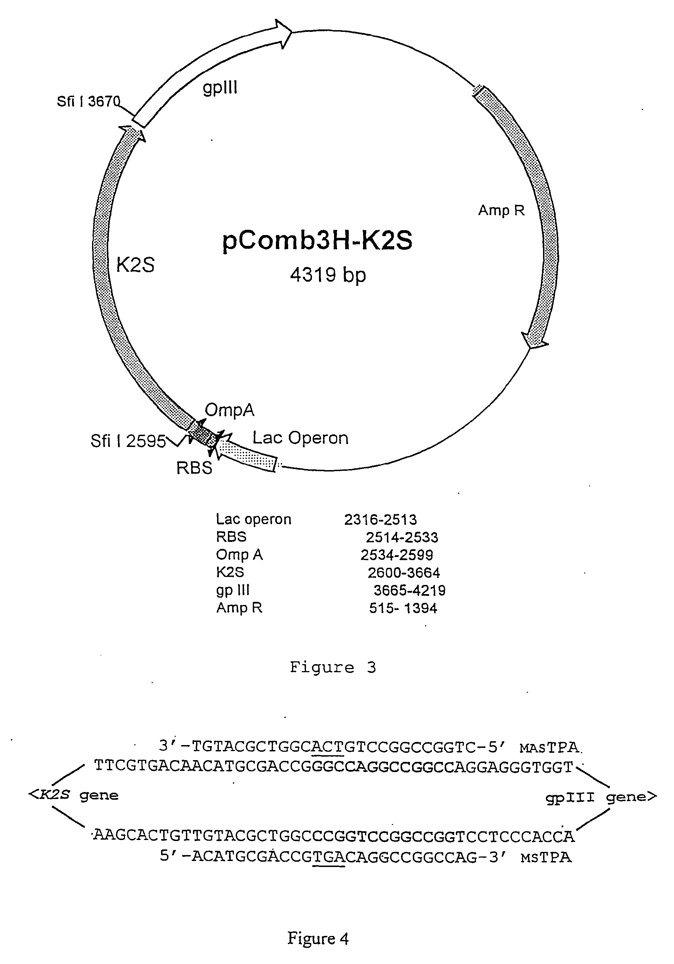

For step a) according to the invention the choice / design of the primers is important to clone the DNA in the right location and direction of the expression vector (see example 1). Thus, the primers as exemplified in example 1 and figure 4 are described. With gp III of step c) gene protein III is meant which is present mainly in phagemid vectors. The stop codon is inserted to avoid transcription of gp III thus eventually leading to secretion of K2S. Any suitable method for insertion of the stop codon may be employed such as site-directed mutagenesis (e.g. Weiner MP, Costa GL (1994) PCR Methods Appl 4(3):S131-136; Weiner MP, Costa GL, Schoettlin W, Cline J, Mathur E, Bauer JC (1994) Gene 151(1-2):119-123; see also example 1).

Any vector may be used in the method according to the invention, preferably said vector is a phagemid vector (see below).

The untranslated region may contain a regulatory element, such as e.g. a transcription initiation unit (promoter) or enhancer. Said promoter may, for example, be a constitutive, inducible or development-controlled promoter. Preferably, without ruling out other known promoters, the constitutive promoters of the human Cytomegalovirus (CMV) and Rous sarcoma virus (RSV), as well as the Simian virus 40 (SV40) and Herpes simplex promoter. Inducible promoters according to the invention comprise antibiotic-resistant promoters, heat-shock promoters, hormone-inducible "Mammary tumour virus promoter" and the metallothioneine promoter. Preferred promotors include T3 promotor, T7 promotor, Lac/aral and Ltet0-1.

More preferably, a method according to the invention is also characterised in that the DNA encoding the heterologous protein is preceeded by a lac promotor and/or a ribosomal binding site such as the Shine-Dalgarno sequence (see also example).

Suitable vectors according to the invention include, but are not limited to viral vectors such as e.g. Vaccinia, Semliki-Forest-Virus and Adenovirus, phagemid vectors and the like. Preferred are vectors which can be advantageously used in E. coli, but also in any other prokaryotic host such as pPROTet.E, pPROLar.A, members of the pBAD family, pSE family, pQE family and pCAL.

Another preferred embodiment of the invention relates to the vector pComb3HSS containing a DNA according to the invention, wherein the expression of the gp III protein is suppressed or inhibited by deleting the DNA molecule encoding said gp III protein or by a stop codon between the gene coding for a a polypeptide containing the heterologous protein and the protein III gene. tPA proteins may include one, several or all of the following domains or subunits or variants thereof:
1. Finger domain (4-50)
2. Growth factor domain (50-87)
3. Kringle 1 domain (87-176)
4. Kringle 2 domain (176-262)
5. Protease domain (276-527)

The numbering/naming of the domains is according to Genbank accession number GI 137119 or Nature 301 (5897), 214-221 (1983).

A method according to the invention is characterised in that the Kringle 2 (4.) plus Serine protease (5.) K2S domain of human tissue plasminogen activator or a functional variant, thereof is produced. More preferably, a method according to the invention is also characterised in that the vector is a phagemid vector comprising the DNA coding for OmpA signal peptide and the DNA coding for gpIII.

The following example is intended to aid the understanding of the invention and should in no way be regarded as limiting the scope of the invention.

### Example 1

### MATERIALS AND METHODS

Primer design. In order to amplify a specific part of tPA gene, a pair of primers _{S}K2/174 [5' GAGGAGGAGGTGGCCCAGGCGGCCTCTGAGGGAAACAGTGAC 3'] (SEQ ID NO:6) and ASSP [5' GAGGAGGAGCTGGCCGGCCTGGCCCGGTCGCATGTTGTCACG 3'] (SEQ ID NO:7) were synthesized (Life Technologies, Grand Island, NY). These primers were designed based on the human tPA gene retrieved from NCBI databases (g137119). They were synthesized with Sfi I end cloning sites (underlined) in such a way that the reading frame from the ATG of the gpIII gene in phagemid vector, pComb3HSS, will be maintained throughout the inserted sequence.

Another primer set for site-directed mutagenesis was designed to anneal at the sequence situated between K2S gene and gene III in pComb3H-K2S. The sequence of primers with mutation bases (underlined) for generating a new stop codon were MSTPA [5' ACATGCGACCGTGACAGGCCGGCCAG 3'] (SEQIDNO:8) and MASTPA [5' CTGGCCGGCCTGTCACGGTCGCATGT 3'] (SEQ ID NO:9).

Amplification of K2S gene by PCR. One µg SK2/174 and ASSP primers together with 50 ng of p51-3 template (obtained from Dr. Hiroshi Sasaki, Fujisawa Pharmaceutical, Japan) were suspended in 100 µl PCR mixture. An amount of 2.5 U Taq polymerase (Roche Molecular Biochemicals, Indianapolis, IN) was finally added to the solution. The titrated amplification condition was initiated with jump start at 85°C for 4 min, then denaturation at 95°C for 50 sec, annealing at 42°C for 50 sec, extension at 72°C for 1.5 min. Thirty five rounds were repeatedly performed. The mixture was further incubated at not for 10 min. The amplified product of 1110 bp was subsequently purified by QIAquick PCR Purification Kit (QIAGEN, Hilden, Germany). The correctness of purified product was confirmed by restriction enzymes.

Construction of phagemid expressing K2S. The purified PCR product of K2S and pComb3HSS phagemid (kindly provided by Dr. Carlos F. Barbas, Scripps Institute, USA) were digested with Sfi I (Roche Molecular Biochemicals, Indianapolis, IN) to prepare specific cohesive cloning sites. Four µg of the purified PCR product was digested with 60 U of Sfi I at 50°C for 18 h. For pComb3HSS, 20 µg of phagemid vectors were treated with 100 U of Sfi I. Digested products of purified PCR product of K2S and pComb3HSS (-3300 bp) were subsequently gel-purified by the QIAquick Gel Extraction Kit (QIAGEN, Hilden, Germany). T4 ligase (Roche Molecular Biochemicals, Indianapolis, IN) of 5 U were introduced to the mixture of 0.7 µg of purified Sfi I-digested pComb3HSS and 0.9 µg of purified SfiI-digested PCR product. Ligation reaction was incubated at 30°C for 18 h. The newly constructed phagemid was named pComb3H-K2S.

Transformation of XL-1 Blue. Two hundred µl of CaCl₂ competent *E. coli* XL-1 Blue (Stratagene, La Jolla, CA) were transformed with 70 ng of ligated or mutated product. The transformed cells were propagated by spreading on LB agar containing 100 µg/ml ampicillin and 10 µg/ml tetracycline (Sigma, Saint Louis, MO). After cultivation at 37°C for 18 h several antibiotic resistant colonies were selected for plasmid minipreps by using the alkaline lysis method. Each purified plasmid was subjected to Sfi I restriction site analysis. A transformant harboring plasmid with the correct Sfi I restriction site(s) was subsequently propagated for 18 h at 37°C in 100 ml LB broth with ampicillin 100 µg/ml and tetracycline 10 µg/ml. A plasmid maxiprep was performed using the QIAGEN Plasmid Maxi Kit (QIAGEN, Hilden, Germany). The purified plasmid was reexamined for specific restriction sites by Sfi I and sequenced by AmpliTaq DNA Polymerase Terminator Cycle Sequencing Kit (The Perkin-Elmer Corporation, Forster City, CA).

Site-directed mutagenesis of pComb3H-K2S. 10 ng of pComb3H-K2S template were mixed with 125 ng of MSTPA and MASTPA primers. PfuTurbo DNA polymerase (Stratagene, LA Jolla, CA) of 2.5 U was added to the mixture for cycle amplification. The reaction started with one round of 95°C for 30 sec. Then it was followed by 16 rounds consisting of 95°C for 30 sec, 55°C for 1 min, and 68°C for 9 min. The reaction tube was subsequently placed on ice for 2 min. In order to destroy the template strands, 10 U of Dpn I restriction enzyme (Stratagene, LA Jolla, CA) were added to the amplification reaction and incubated for 1 h at 37°C. This synthesized product (MpComb3H-K2S) was further used to transform *E*. *coli* XL-1 Blue.

Preparation of phage-display recombinant-K2S. After pComb3H-K2S was transformed to *E*. *coli* XL-1 Blue, the phage display technique was performed. A clone of pComb3H-K2S transformed *E*. *coli* XL-1 Blue was propagated in 10 ml super broth containing ampicillin 100 µg/ml and tetracycline 10 µg/ml at 37°C until the O.D. [600 nm] of 1.5 was reached. The bacterial culture was subsequently propagated in 100 ml of the same medium and culture for 2 h. An amount of 10¹² pfu of VCSM13 helper phage (Stratagene, La Jolla, CA) was used to infect the transformed *E*. *coli* XL-1 Blue. After 3 h incubation, kanamycin at a final concentration of 70 µg/ml final concentration was added to culture. The culture was left shaking (200 RPM) for 18 h at 37°C. Bacteriophages which harbored K2S on gp3 (K2S-φ) were then harvested by adding 4% w/v PEG MW 8000 (Sigma, Saint Louis, MO) and 3% w/v NaCl. Finally, the harvested phage was resuspended in 2 ml PBS pH 7.4. The phage number was determined by infecting *E*. *coli* XL-1 Blue. The colony-forming unit per milliliter (cfu/ml) was calculated as described previously (21).

Expression of recombinant-K2S in shaker flasks. MpComb3H-K2S transformed XL-1 Blue was cultivated in 100 ml super broth (3% w/v tryptone, 2% w/v yeast extract and 1% w/v MOPS) at pH 7.0 in the presence of ampicillin (100 µg/ml) at 37°C until an O.D. [600 nm] of 0.8 was reached. Subsequently, the protein synthesis was induced by 1 mM of IPTG (Promega, Madison, WI). The bacteria were further cultured shaking (200 RPM) for 6 h at 30°C. The culture supernatant was collected and precipitated with 55% saturated ammonium sulfate (32). The precipitate was reconstituted with PBS, pH 7.2, and dialysed in the same buffer solution at 4°C for 18 h. Periplasmic proteins from bacterial cells were extracted by using a chloroform shock as previously described by Ames et al. (2).

Immunoassay quantification of recombinant-K2S. In order to detect r-K2S, solid phase was coated with monoclonal anti-kringle 2 domain (16/B) (generously provided by Dr. Ute Zacharias, Central Institute of Molecular Biology, Berlin-Buch, Germany). The standard ELISA washing and blocking processes were preformed. Fifty µl of 10¹¹ cfu/ml of K2S-φ or secretory r-K2S were added into each anti-kringle 2 coated well. Antigen-antibody detection was carried out as follows. Either sheep anti-M13 conjugated HRP (Pharmacia Biotech, Uppsala, Sweden) or sheep anti-tPA conjugated HRP (Cedarlane, Ontario, Canada), was added to each reaction well after the washing step. The substrate TMB was subjected to every well and the reaction was finally ceased with H₂SO₄ solution after 30 min incubation. The standard melanoma tPA 86/670 (National Institute for Biological Standards and Control, Hertfordshine, UK) was used as positive control.

Amidolytic activity assay. A test kit for the detection of tPA amidolytic activity was purchased from Chromogenix (Molndal, Sweden). The substrate mixture containing plasminogen and S-2251 was used to determine serine protease enzymatic activity. The dilution of 10⁻² of each ammonium precipitated sample was assayed with and without stimulator, human fibrinogen fragments. The assay procedure was according to the COASET t-PA manual.

SDS-PAGE and immunoblotting. The dialysed precipitate-product firm culture supernatant was further concentrated 10 folds with centricon 10 (AMICON, Beverly, MA). The concentrated sample was subjected to protein separation by SDS-PAGE, 15% resolving gel, in the reducing buffer followed by electroblotting to nitrocellulose. The nitrocellulose was then blocked with 4% skimmed milk for 2 hr. In order to detect r-K2S, a proper dilution of sheep anti-tPA conjugated HRP was applied to the nitrocellulose. The immunoreactive band was visualized by a sensitive detection system, Amplified Opti-4CN kit (BIORAD, Hercules, CA).

Copolymerized plasminogen polyacrylamide gel electrophoresis. An 11% resolving polyacrylamide gel was copolymerized with plasminogen and gelatin as previously described by Heussen et al. (14). The stacking gel was prepared as 4 % concentration without plasminogen and gelatin. Electrophoresis was performed at 4°C at a constant current of 8 mA. The residual SDS in gel slab was removed after gentle shaking at room temperature for 1h in 2.5% Triton X-100. Then the gel slab was incubated in 0.1 M glycine-NaOH, pH 8.3, for 5 h at 37°C. Finally, the gel slab was stained and destained by standard Coomassie brilliant blue (R-250) dying system. The location of the peptide harboring enzymatic activity was not stained by dye in contrast to blue-paint background.

### RESULTS

Construction of K2S gene carrying vector. From the vector p51-3 we amplified the kringle 2 plus ther serine protease portion of tPA (Ser¹⁷⁴ in kringle 2 domain to Pro⁵²⁷ in the serine protease) using primers SK2/174 and ASSP. The amplified 1110 bp product was demonstrated by agarose gel electrophoresis (Fig. 1, lane 2) and was inserted into pComb3HSS phagemid by double Sfi I cleavage sites on 5' and 3' ends in the correct reading frame. Thus a new vector, pComb3H-K2S, harboring the K2S was generated. In this vector K2S is flanked upstream by the OmpA signal sequence and donwstream by gp3. The correct insertion of K2S was verified both by restriction analysis with Sfi I (Fig. 2, lane 3), PCR-anaysis (demonstration of a single band at 1110 bp), and DNA sequencing. The schematic diagram of pComb3H-K2S map is given in Fig. 3.

Phage-displayed r-K2S. VCSM13 filamentous phage was used to infect pComb3H-K2S transformed *E*. *coli* XL-1 Blue, X[K2S]. VCSM13 was propagated and incorporated the K2S-gp3 fusion protein during the viral packaging processes. The harvested recombinant phage (K2S-φ) gave a concentration of 5.4 x 10¹¹ cfu/ml determined by reinfecting *E*. *coli* XL-1 Blue with PEG-precipitated phages. These recombinant phage particles were verified for the expression of r-K2S by sandwich ELISA. The phage-bound heterologous K2S protein was recognized by the monoclonal anti-kringle 2 antibody (16/B) by using sheep anti-tPA conjugated HRP antibody detection system. The absorbance of this assay was I.12 ± 0.03 (Table 1). The amount of K2S detectable on 10¹² phage particles is equal to 336 ng of protein in relation to the standard melanoma tPA. In order to corroborate that K2S-gp3 fusion protein was associated with phage particles, sheep anti-tPA conjugated HRP antibody was substituted by sheep anti-M13 antibody conjugated HRP. This immuno-reaction exhibited an absorbance of 1.89 ± 0.07 (Table 1). In contrast, if the capture antibody was sheep anti-M13 antibody, extremely low K2S was observed with sheep anti-tPA antibody conjugated HRP; the absorbance was only 0.17 ± 0.01 (Table 1). This suggested that only a minority of purified phage particles carried K2S-gp3 fusion protein. VCSM13 prepared from non-transformed *E*. *coli* XL-1 Blue was used as a negative control.

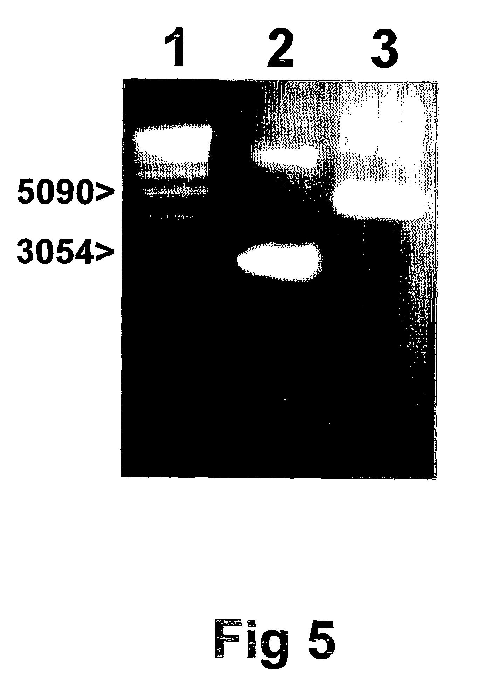

Construction of MpComb3H-K2S. We generated a stop codon between K2S and gp3 in pComb3H-K2S with the aid of the mutagenic primers (MSTPA and MASTPA) (Fig. 4). In order to enrich the newly synthesized and mutated MpComb3H-K2S, the cycle amplification mixture was thoroughly digested with Dpn I to degrade the old dam methylated pComb3H-K2S template (Dpn I prefers dam methylated DNA). After transforming of *E*. *coli* XL-1 Blue with MpComb3H-K2S, a transformant XM[K2S] was selected for further study. As a consequence of bp substitution, one Sfi I cleavage site close to the 3' end of K2S gene was lost after site-directed mutagenesis. A linear version of Sfi I cleaved MpComb3H-K2S was observed at 4319 bp without the appearance of inserted K2S gene fragment (Fig. 5, lane 3). Thus, the K2S gene encoding by MpComb3H-K2S was expressed in non-gp3 fusion form in *E*. *coli* XM[K2S].

Expression and purification of K2S. K2S expression in *E*. *coli* XM[K2S] was induced by IPTG. r-K2S was detectable by using ELISA both in the periplasmic space and in the culture supernatant. The amount of the heterologous protein in each preparation was determined by sandwich ELISA and related to the standard tPA. From 100 ml of the bacterial culture in shaker flask with the O.D. [600 nm] of 50, the periplasmic fraction yielded 1.38 µg of r-K2S (approximately 32%) whereas 2.96 µg of r-K2S (approximately 68%) was obtained in the ammonium precipitated culture supernatant. Sandwich ELISA was used to verify the PEG precipitated phage from VCSM13 infected *E*. *coli* XM[K2S]. No r-K2S captured by monoclonal anti-kringle 2 antibody was detected by anti-M13 conjugated HRP, indicating that K2S is not presented on the phage particles if gp3 is missing.

Amidolytic activity measurement. If serine protease domain is present in the sample, plasminogen will be converted to plasmin. The produced plasmin will further digest the S-2251 substrate to a colour product, p-nitroaniline, which has a maximum absorbance at 405 nm. The specific activity of the recombinant product is in accord with the absorbance. The fibrinogen-dependent enzymatic activity of each sample i.e. K2S-φ, periplasmic r-K2S or culture supernatant r-K2S, was evaluated and compared. Both K2S-φ and periplasmic r-K2S illustrated notably low enzymatic activity, which was below the sensitivity of the test (0.25 IU/ml). The culture supernatant r-K2S gave the fibrinogen-dependent enzymatic activity of 7 IU/ml. Thus, from 100 ml culture we obtained a total of 700 IU enzymatic activity. Without fibrinogen no enzymatic activity of the r-K2S purified from culture supernatant was observed - whereas standard melanoma tPA showed some activity.

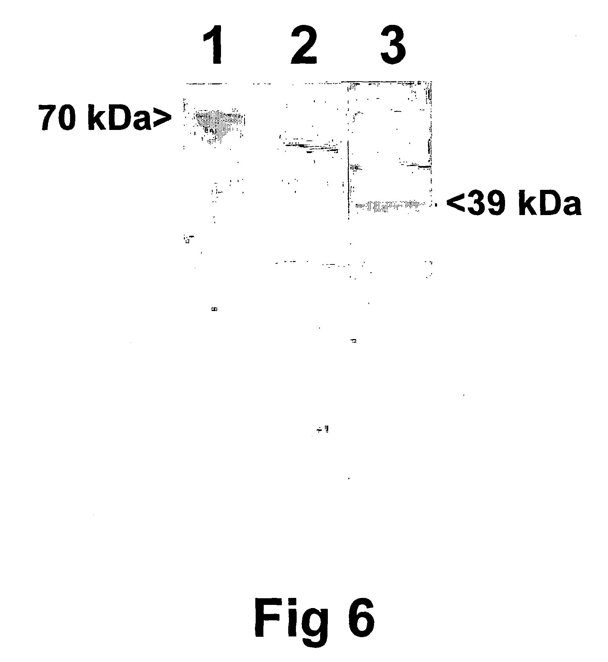

Demonstration of recombinant protein by immunoblotting. Partially purified K2S from culture supernatant of *E*. *coli* XM[K2S] revealed a molecular mass of 39 kDa by using sheep anti-tPA antibodies (Fig. 6). The negative control, partially purified culture supernatant of non-transformed *E*. *coli* XL1-Blue, contained no reactive band with a similar size.

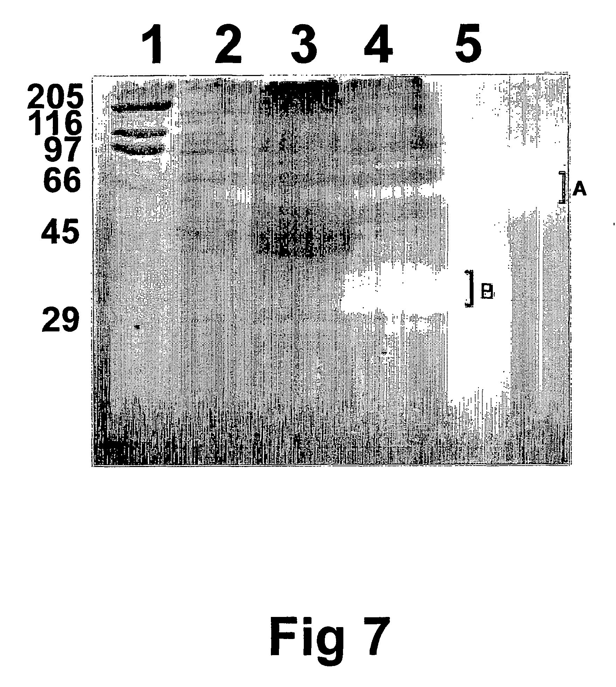

Localization of active enzyme by PAGE. The plasminogen has been copolymerized and immobilized with gelatin in the polyacrylamide gel prior to electrophoresis. The ammonium sulfate precipitated culture supernatants of *E*. *coli* XL-1 Blue, *E*. *coli* XL-1 Blue transformed with pComb3HSS and *E*. *coli* XM[K2S] were analyzed (Fig. 7). All samples were processed in non-reducing condition to preserve the correct conformation and activity of the serine protease domain. Transparent areas of serine protease digested plasminogen were observed only in the ammonium sulfate precipitated culture supernatants of *E*. *coli* XM[K2S] at 34 and 37 kDa postions. The other samples gave no clearing zones. The positive control lane of standard melanoma tPA also demonstrated enzymatic activity at 66 and 72 kDa positions.

### References

1. Allen, S., H. Y. Naim, and N. J. Bulleid. 1995. Intracellular folding of tissue-type plasminogen activator. Effects of disulfide bond formation on N-linked glycosylation and secretion. J. Biol. Chem. 270:4797-4804.
2. Ames, G. F., C. Prody, and S. Kustu. 1984. Simple, rapid, and quantitative release of periplasmic proteins by chloroform. J. Bacteriol. 160:1181-1183.
3. Barbas, C. F. III, A. S. Kang, R. A. Lerner, and S. J. Benkovic. 1991. Assembly of combinatorial antibody libraries on phage surfaces: the gene III site. Proc. Natl. Acad. Sci. U. S. A. 88:7978-7982.
4. Barbas, C. F. III, and J. Wagner. 1995. Synthetic human antibodies: selecting and evolving functional proteins. A Companion to Methods in Enzymology 8: 94-103.
5. Bennett, W. F., N. F. Paoni, B. A. Keyt, D. Botstein, A. J. Jones, L. Presta, F. M. Wurm, and M. J. Zoller. 1991. High resolution analysis of functional determinants on human tissue-type plasminogen activator. J Biol Chem. 266:5191-5201.
6. Betton, J. M., N. Sassoon, M. Hofnung, and M. Laurent. 1998. Degradation versus aggregation of misfolded maltose-binding protein in the periplasm of Escherichia coli. J. Biol. Chem. 273:8897-8902.
7. Camiolo, S. M., S. Thorsen, and T. Astrup. 1971. Fibrinogenolysis and fibrinolysis with tissue plasminogen activator, urokinase, streptokinase-activated human globulin and plasmin. Proc. Soc. Exp. Biol. Med. 38:277-280.
8. Cartwright, T. 1992. Production of t-PA from animal cell culture, p. 217-245. In R. E. Spier, and J. B. Griffiths (ed.), Animal Cell Biotechnology, Vol 5. Academic Press, N.Y.
9. Curry, K. A., A. W. Yem, M. R. Deibel, Jr., N. T. Hatzenbuhler, J. G. Hoogerheide, and C. S. Tomich. 1990. Escherichia coli expression and processing of human interleukin-1 beta fused to signal peptides. DNA Cell Biol. 9:167-175.
10. Datar, R. V., T. Cartwright, an C.-G. Rosen. 1993. Process economics of animal cell and bacterial fermentations: a case study analysis of tissue plasminogen activator. Biotechnology 11:349-357.
11. Denefle, P., S. Kovarik, T. Ciora, N. Gosselet, J. C. Benichou, M. Latta, F. Guinet, A. Ryter, and J. F. Mayaux. 1989. Heterologous protein export in Escherichia coli: influence of bacterial signal peptides on the export of human interleukin 1 beta. Gene 85:499-510.
12. Griffiths, J. B., A. Electricwala. 1987. Production of tissue plasminogen activators from animal cells. Adv. Biochem. Eng. Biotechnol. 34:147-166.
13. Harris, T. J., T. Patel, F. A. Marston, S. Little, J. S. Emtage, G. Opdenakker, G. Volckaert, W. Rombauts , A. Billiau, and P. De Somer. 1986. Cloning of cDNA coding for human tissue-type plasminogen activator and its expression in Escherichia coli. Mol. Biol. Med. 3:279-292.
14. Heussen, C., and E. B. Dowdle. 1980. Electrophoretic analysis of plasminogen activators in polyacrylamide gels containing sodium dodecyl sulfate and copolymerized substrates. Anal. Biochem. 102:196-202.
15. Heussen, C., F. Joubert, and E. B. Dowdle. 1984. Purification of human tissue plasminogen activator with Erythrina trypsin inhibitor. J. Biol. Chem. 259:11635-11638.
16. Hu, C. K., U. Kohnert, O. Wilhelm, S. Fischer, and M. Llinas. 1994. Tissue-type plasminogen activator domain-deletion mutant BM 06.022: modular stability, inhibitor binding, and activation cleavage. Biochemistry 33:11760-11766.
17. Kipriyanov, S. M., G. Moldenhauer, and M. Little. 1997. High level production of soluble single chain antibodies in small-scale Escherichia coli cultures. J. Immunol. Methods 200:69-77.
18. Ko, J. H., D. K. Park, I. C. Kim, S. H. Lee, and S. M. Byun. 1995. High-level expression and secretion of streptokinase in Excherichia coli. Biotechnol. Lett. 17:1019-1024.
19. Kouzuma, Y., N. Yamasaki, and M. Kimura. 1997. The tissue-type plasminogen activator inhibitor ETIa from Erythrina variegata: structural basis for the inhibitory activity by cloning, expression, and mutagenesis of the cDNA encoding ETIa. J. Biochem. (Tokyo) 121:456-463.
20. Lasters, I., N . Van Herzeele, H. R Lijnen, D. Collen, and L. Jespers. 1997. Enzymatic properties of phage-displayed fragments of human plasminogen. Eur. J. Biochem. 244:946-952.
21. Lobel, L. I., P. Rausch, I. Trakht, S. Pollak, and J. W. Lustbader. 1997. Filamentous phage displaying the extracellular domain of the hLH/CG receptor bind hCG specifically. Endocrinology. 138:1232-1239.
22. Lubiniecki, A., R. Arathoon, G. Polastri, J. Thomas, M. Wiebe, R. Gamick, A. Jones, R van Reis, and S. Builder. Selected strategies for manufacture and control of recombinant tissue plasminogen activator prepared from cell culture, p. 442-451. In R E. Spier, J. B. Griffiths, J. Stephenne, and P. J. Crooy (ed.), Advances in animal cell biology and technology for bioprocesses. Butterworths, London.
23. Lucic, M. R., B. E. Forbes, S. E. Grosvenor, J. M. Carr, J. C. Wallace, and G. Forsberg. 1998. Secretion in Escherichia coli and phage-display of recombinant insulin-like growth factor binding protein-2. J. Biotechnol. 61:95-108.
24. Martin, U., S. Fischer, U. Kohnert, H. Lill, R. Rudolph, G. Sponer, A. Stem, and K. Strein. 1990. Properties of a novel plasminogen activator (BM 06.022) produced in Escherichia coli. Z. Kardiol. 79:167-170.
25. Obukowicz, M. G., M. E. Gustafson, K. D. Junger, R. M. Leimgruber, A. J. Wittwer, T. C. Wun, T. G. Warren, B. F. Bishop, K. J. Mathis, D. T. McPherson, N. R. Siegel, M. G. Jenning, B. B. Brightwell, J. A. Diaz-Cllier, L. D. Bell, C. S. Craik, and W. C. Tacon. 1990. Secretion of active kringle-2-serine protease in Escherichia coli. Biochemistry 29:9737-9745.
26. Parmley, S. F., and G. P. Smith. 1988. Antibody-selectable filamentous fd phage vectors: affinity purification of target genes. Gene 73:305-318.
27. Pennica, D., W. E. Holmes, W. J. Kohr, R N. Harkins, G. A. Vehar, C. A. Ward, W. F. Bennett, E. Yelverton, P. H. Seeburg, H. I. Heyneker, D. V. Goeddel, and D. Collen. 1983. Cloning and expression of human tissue-type plasminogen activator cDNA in E. coli. Nature 301:214-221.
28. Rippmann, J. F., M. Klein, C. Hoischen, B. Brocks, W. J. Rettig, J. Gumpert, K. Pfizenmaier, R. Mattes, and D. Moosmayer. 1998. Procaryotic expression of single-chain variable-fragment (scFv) antibodies: secretion in L-form cells of Proteus mirabilis leads to active product and overcomes the limitations of periplasmic expression in Escherichia coli. Appl. Environ. Microbiol. 64:4862-4869.
29. Saito, Y., Y. Ishii, H. Sasaki, M. Hayashi, T. Fujimura, Y. Imai, S. Nakamura, S. Suzuki, J. Notani, T. Asada, H. Horiai, K. Masakazu, and N. Mineo. 1994. Production and characterization of a novel tissue-type plasminogen activator derivative in Escherichia coli. Biotechnol. Prog. 10:472-479.
30. Sarmientos, P., M. Duchesne, P. Denèfle, J. Boiziau, N. Fromage, N. Delporte, F. Parker, Y. Lelièvre, J.-F. Mayaux, and T. Cartwright. 1989. Synthesis and purification of active human tissue plasminogen activator from Escherichia coli. Biotechnology 7:495-501.
31. Scherrer, S., N. Robas, H. Zouheiry, G. Branlant, and C. Branlant. 1994. Periplasmic aggregation limits the proteolytic maturation of the Escherichia coli penicillin G amidase precursor polypeptide. Appl. Microbiol. Biotechnol. 42:85-89.
32. Soeda, S., M. Kakiki, H. Shimeno, and A. Nagamatsu. 1986. Rapid and high-yield purification of porcine heart tissue-type plasminogen activator by heparin-sepharose choromatography. Life Sci. 39:1317-1324.
33. Szarka, S. J., E. G. Sihota, H. R. Habibi., and S.-L. Wong. 1999. Staphylokinase as a plasminogen activator component in recombinant fusion proteins. Appl. Environ. Microbiol. 65:506-513.
34. Waldenström, M., E. Holmgren, A. Attersand, C. Kalderen, B. Lowenadler, B. Raden, L. Hansson, and G. Pohl. 1991. Synthesis and secretion of a fibrinolytically active tissue-type plasminogen activator variant in Escherichia coli. Gene 99:243-248.
35. Wan, E. W.-M., and F. Baneyx. 1998. TolAIII Co-overexpression Facilitates the Recovery of Periplasmic Recombinant Proteins into the Growth Medium of Escherichia coli. Protein Expr. Purif. 14:13-22.
36. Zacharias, U., B. Fischer, F. Noll, and H. Will. 1992. Characterization of human tissue-type plasminogen activator with monoclonal antibodies: mapping of epitopes and binding sites for fibrin and lysine. Thromb. Haemost. 67:88-94.
37. Hua Z-C, Fu H-L, Chen Y-H, Yu R-R, Wang J and Zhu D-X. 1994. Synthesis and expression of a gene from kringle-2 domain of tissue plasminogen activator in E. coli. Science in China 37:667-676.

### Figure Legends

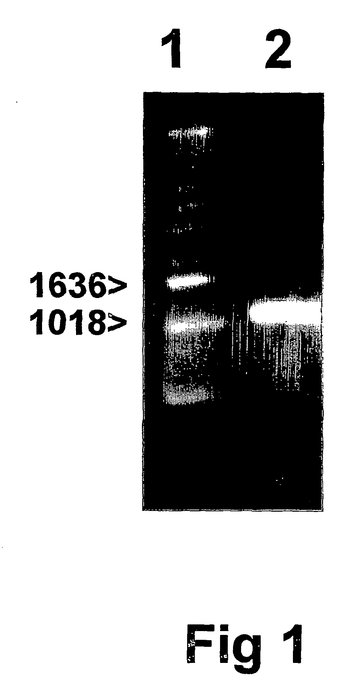

FIG. 1. Validation of PCR amplification product of the K2S gene from the p51-3 vector by using SK2/174 and ASSP primers. Lane 1 shows 1 kb marker (Roche Molecular Biochemicals, Indianapolis, IN). Lane 2 was loaded with 1 µl of amplified product. A single band at 1110 bp is depicted. The electrophoresis was performed on a 1% agarose gel.

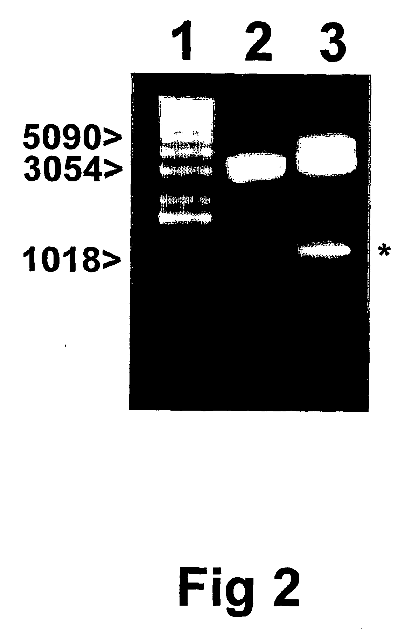

FIG. 2. Identification of inserted K2S gene at 1110 bp (*) after Sfi I digested pComb3H-K2S was demonstrated in lane 3. Lane 1 shows 1 kb marker. Lane 2 was loaded with uncut pComb3H-K2S. The electrophoresis was performed on a 1% agarose gel.
FIG. 3. Scheme of pComb3H-K2S showing two Sfi I cloning sites into which the K2S gene was inserted. Signal sequence (OmpA), ribosome binding site (RIBS), lac promotor, and gpIII gene are also depicted.
FIG. 4. Schematic diagram of the mutation site at the junction between the K2S and gpIII genes on pComb3H-K2S. The annealing site of pComb3H-K2S is bound with a set of mutation primers (MSTPA and MASTPA) containing modified oligonucleosides (underlined). After performing the cycle amplification, the Sfi I site 1 (in bold) is modified and lost in the newly synthesized strand.
FIG. 5. Characterization of newly synthesized MpComb3H-K2S by the Sfi I restriction enzyme. A single band at 4319 bp that refers to a single cleavage site of MpComb3H-K2S is observed in lane 3. No inserted K2S band at 1110 bp can be visualized. Lane 1 shows 1 kb marker. Lane 2 was loaded with uncut MpComb3H-K2S. The electrophoresis was performed on a 1% agarose gel.
FIG. 6. Identification of immunological reactive band with of recombinant protein purified from *E*. *coli* XM[K2S] culture supernatant with sheep anti-tPA conjugated HRP. Lane 1 was loaded with 40 ng of standard melanoma tPA (86/670), which showed the reactive band at 70 kDa. The partially purified and concentrated culture supernatants from non-transformed *E*. *coli* XL1- Blue *and E*. *coli* XM[K2S] were applied to lane 2 and 3 respectively. The distinct reactive band was particularly demonstrated in lane 3 at 39 kDa.
FIG. 7. Molecular weight determination of extracellular r-K2S harboring active serine protease domain by copolymerized plasminogen polyacrylamide gel electrophoresis. Lane 1 contained the indicated molecular weight standards (× 10⁻³), SDS-6H (Sigma, Saint Louis, MO). Fifty µg of the 55% saturated ammonium sulfate precipitated culture supernatant of *E*. *coli* Z-1 Blue, *E*. *coli* X1-1 Blue transformed with pComb3HSS, and *E*. *coli* XM[K2S] were loaded in lane 2, 3, and 4 respectively. Lane 5 contained 50 mIU of standard melanoma tPA (86/670). Transparent zones of digested plasminogen in polyacrylamide gel are visible only in lane 4 at molecular weight of 34 and 37 kDa (B) and lane 5 at molecular weight of 66 and 72 kDa (A).

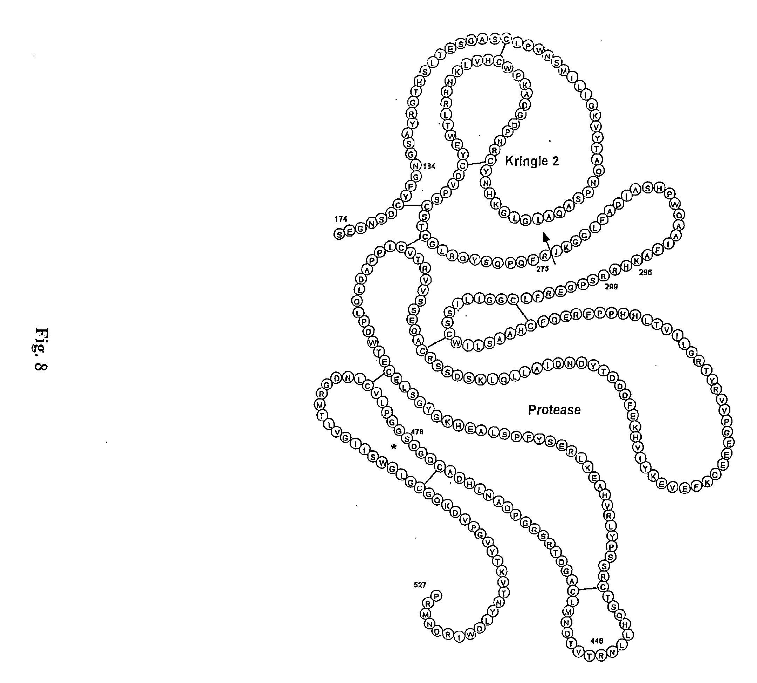

FIG. 8. Structure A
   Native K2S molecule from amino acids 174-527 without modification (SEQ ID NO:10).

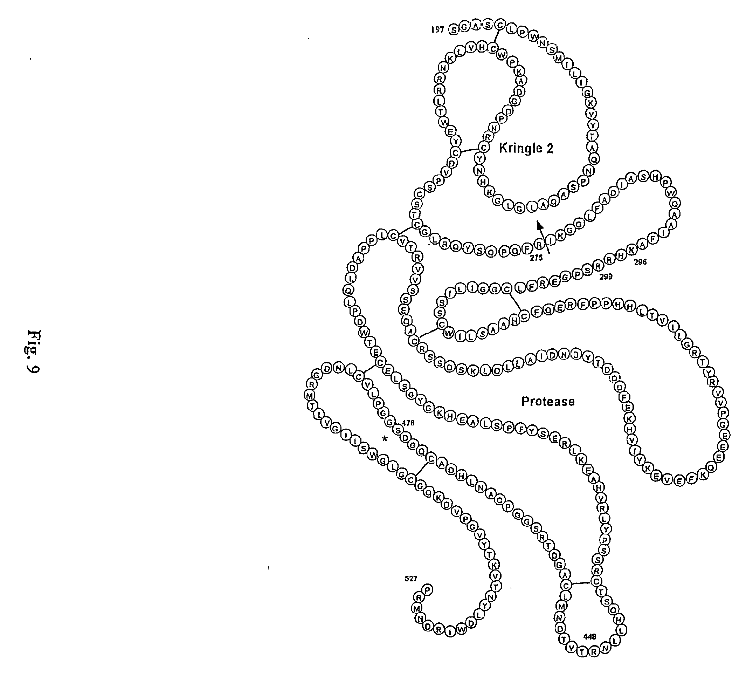

FIG. 9. Structure B-0
   Native K2S molecule from amino acids 197-527 without modification. (SEQ ID NO:11)

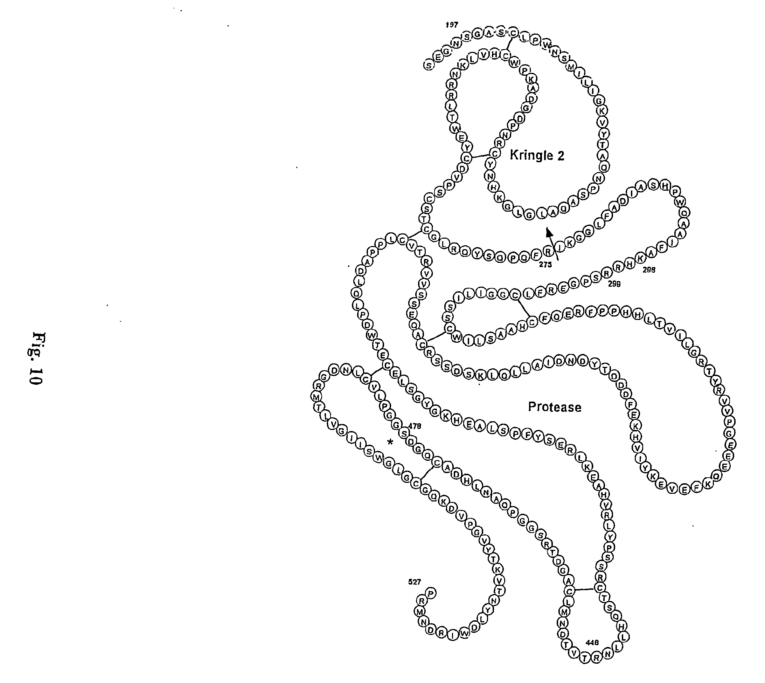

FIG. 10. Structure B-1
   K2S molecule from amino acids 193-527, wherein to Structure B-0 of Fig. 9 the amino acids SEGN were added at the N-terminal portion (SEQ ID NO:12).

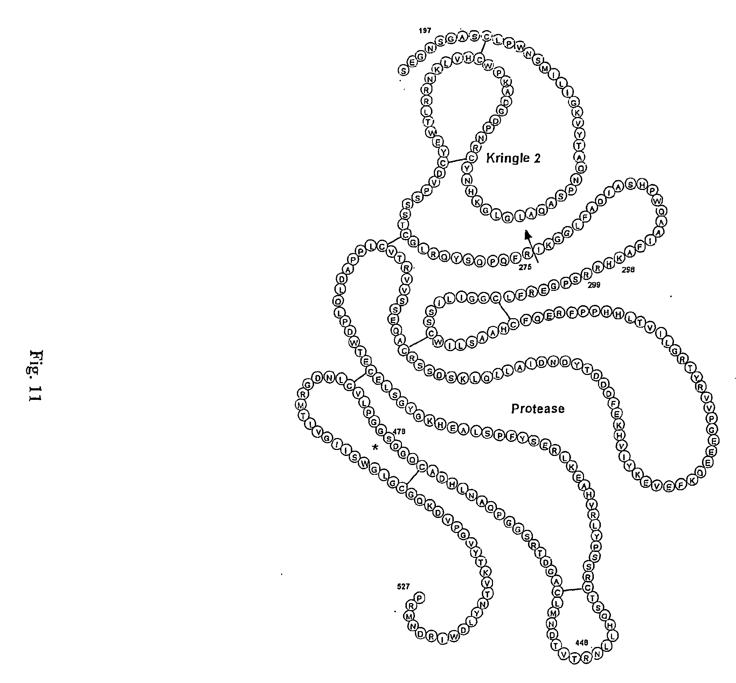

FIG. 11. Structure B-2
   K2S molecule from amino acids 193-527, as in Fig. 10, wherein Cys-261 was exchanged for Ser (SEQ ID NO: 13).

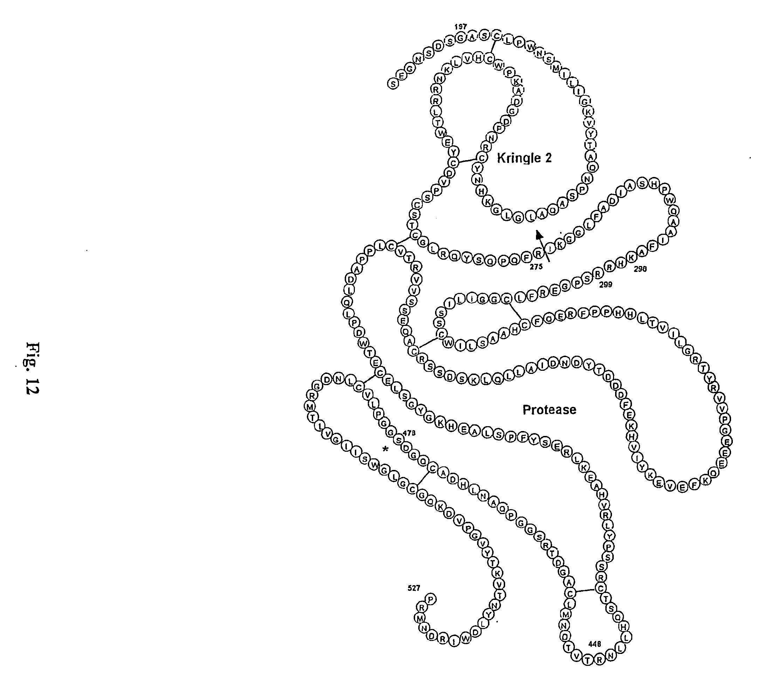

FIG. 12. Structure B-3
   K2S molecule from amino acids 191-527, wherein to Structure B-0 of Fig. 9 the amino acids SEGNSD were added at the N-terminal portion (SEQ ID NO:14).

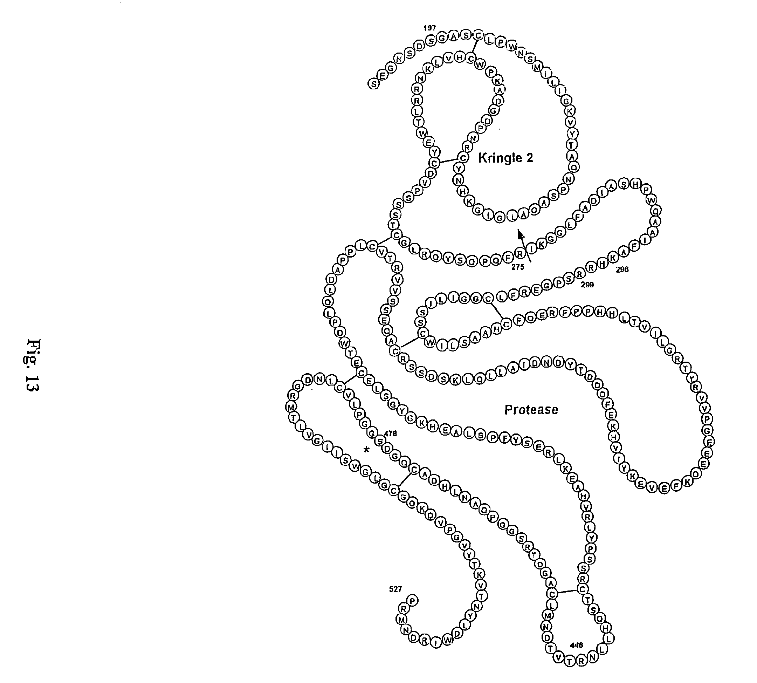

FIG. 13. Structure B-4
   K2S molecule from amino acids 191-527, as in Fig. 12, wherein Cys-261 was exchanged for Ser (SEQ ID NO:15).

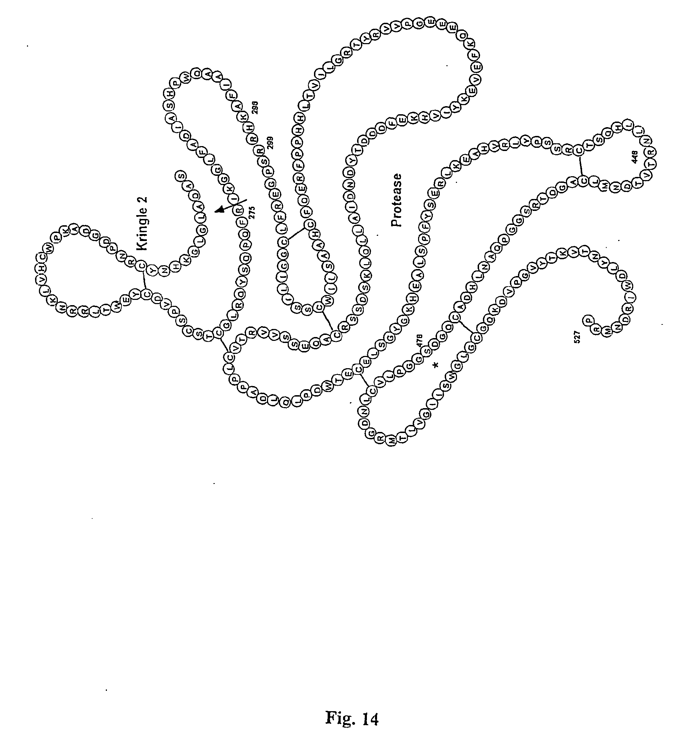

FIG. 14. Structure C
   Native K2S molecule from amino acids 220-527 without modification. This molecule may be further modified in a similar manner as disclosed for structure B in figures 10-13 (SEQ ID NO:16).

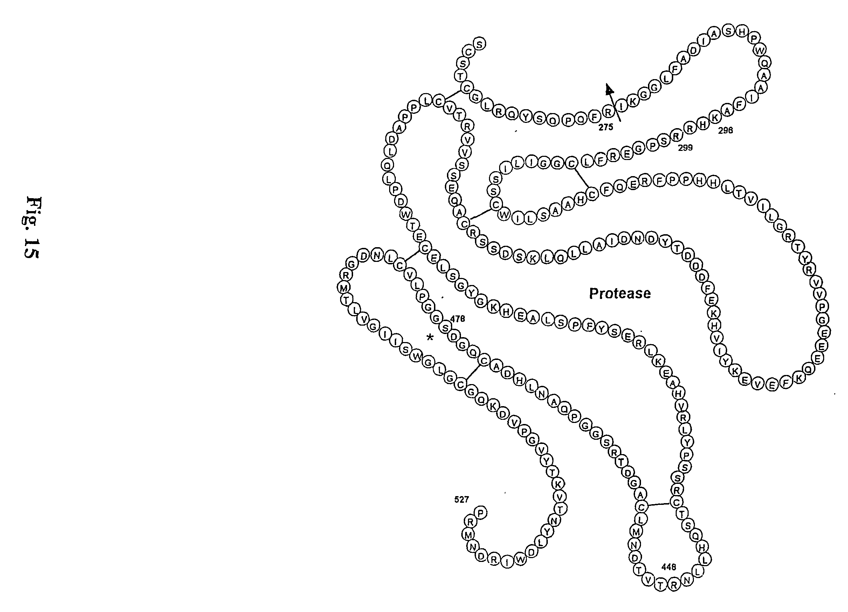

FIG. 15. Structure D
   Native K2S molecule from amino acids 260-527 without modification. This molecule may be further modified in a similar manner as disclosed for structure B in figures 10-13 (SEQ ID NO: 17).

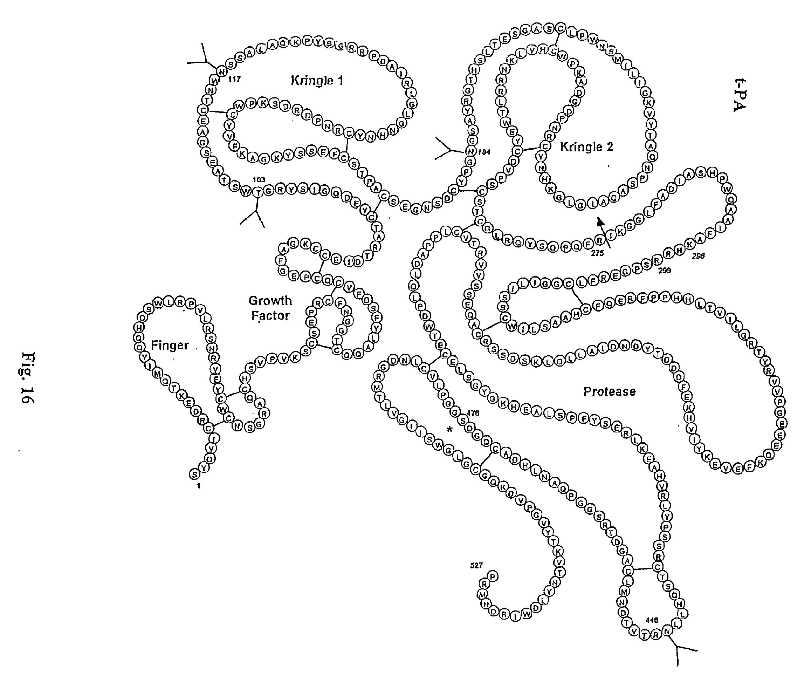

FIG. 16. tPA molecule (SEQ ID NO:18)

### Table

**TABLE 1. Detection of r-K2S molecule in phage preparation by sandwich ELISA**

| Capture antibody | Tracer antibody (conjugated HRP) | | | |
|---|---|---|---|---|
| | Anti-tPA | | Anti-M13 | |
| | K2S-φ | VCSM13^{a} | K2S-φ | VCSM13 |
| Anti-kringle 2^{b} | 1.12 ± 0.04^{c} | 0.12 ± 0.03 | 1.89 ± 0.02 | 0.16 ± 0.02 |
| Anti-M13 | 0.17 ± 0.01 | 0.14 ± 0.05 | 1.91 ± 0.02 | 1.88 ± 0.03 |

| | | | | |
|---|---|---|---|---|
| ^{a} VCSM13 was harvested from XL-1 Blue transformed with pComb3HSS. ^{b} Mouse monoclonal anti-kringle 2 (16/B) was used. The other antibodies were prepared from sheep immunoglobulin. ^{c} Value is mean of absorbance of each sample which was assayed in triplicate. | | | | |

## Claims

1. Method for the production of active and correctly folded Kringle 2 plus Serine protease domain (K2S) of human tissue plasminogen activator or a functional variant thereof in the supernatant of prokaryotic cells, **characterized in that** the prokaryotic cell contains and expresses a vector comprising a DNA coding for the signal peptide of outer membrane protein A (OmpA), which is downstream fused to a DNA coding for a short peptide **characterized by** the amino acid sequence SEGNSD (SEQ ID NO:3), which itself is downstream fused to a DNA coding for the Kringle 2 plus Serine protease domain of human tissue plasminogen activator or a functional variant thereof.

2. Method according to claim 1, **characterised in that** the prokaryotic cell is E. coli.

3. Method according to claim 1 or 2, **characterised in that** the following steps are carried out:
a) the DNA encoding the Kringle 2 plus Serine protease domain is amplified by PCR;
b) the PCR product is purified;
c) said PCR product is inserted into a vector comprising the DNA coding for OmpA signal peptide and the DNA coding for gpIII in such a way that said PCR product is operably linked upstream to the DNA coding for the OmpA signal sequence and linked downstream to the DNA coding for gpIII of said vector;
d) that a stop codon is inserted between said Kringle 2 plus Serine protease domain and gpIII;
e) said vector is expressed by the prokaryotic cell;
f) the Kringle 2 plus Serine protease domain protein is purified.

4. Method according to one of claims 1 to 3, **characterised in that** the vector is a phagemid vector comprising the DNA coding for OmpA signal peptide and the DNA coding for gpIII.

5. Method according to one of claims 1 to 4, **characterised in that** the vector is the pComb3HSS phagemid.

6. Method according to one of claims 1 to 5, **characterised in that** the DNA Sequence of OmpA consists of the following sequence:

7. Method according to one of claims 1 to 6, **characterised in that** the DNA coding for the signal peptide of outer membrane protein A (OmpA), which itself is downstream fused to a DNA coding for a short peptide **characterized by** the amino acid sequence SEGNSD (SEQ ID NO:3), which itself is downstream fused to a DNA coding for the Kringle 2 plus Serine protease domain of human tissue plasminogen activator or a functional variant thereof is preceeded by a lac promotor and/or a ribosomal binding site.

## Patentansprüche

1. Verfahren zur Herstellung einer aktiven und korrekt gefalteten Kringle 2-plus-serin-Protease-Domäne (K2S) von humanem Gewebe-Plasminogenaktivator oder einer funktionellen Variante davon im Überstand von prokaryontischen Zellen, **dadurch gekennzeichnet, dass** die prokaryontische Zelle einen Vektor enthält und exprimiert, der eine für ein Signalpeptid des äußeren Membranproteins A (OmpA) kodierende DNA umfasst, die strangabwärts mit einer für ein kurzes Peptid, das durch die Aminosäuresequenz SEGNSD (SEQ ID NO:3) charakterisiert ist, kodierenden DNA fusioniert ist, die ihrerseits strangabwärts mit einer DNA fusioniert ist, die für die Kringle 2-plus-Serin-Protease-Domäne von humanem Gewebe-Plasminogenaktivator oder einer funktionellen Variante davon kodiert.

2. Verfahren nach Anspruch 1, **dadurch gekennzeichnet, dass** es sich bei der prokaryontischen Zelle um E. coli handelt.

3. Verfahren nach Anspruch 1 oder 2, **dadurch gekennzeichnet, dass** die folgenden Stufen durchgeführt werden:
a) die DNA, die für die Kringle 2-plus-Serin-Protease-Domäne kodiert, wird durch PCR amplifiziert;
b) das PCR-Produkt wird gereinigt;
c) dieses PCR-Produkt wird so in einen Vektor, der die für das OmpA-Signalpeptid kodierende DNA und die für gpIII kodierende DNA umfasst, inseriert, so dass das PCR-Produkt funktionell strangaufwärts mit der für die OmpA-Signalsequenz kodierenden DNA verknüpft ist und strangabwärts mit der für gpIII kodierenden DNA dieses Vektors verknüpft ist;
d) ein Stoppkodon wird zwischen der Kringle 2-plus-Serin-Protease-Domäne und gpIII inseriert;
e) dieser Vektor wird durch die prokaryontische Zelle exprimiert;
f) das Kringle 2-plus-Serin-Protease-Domäne-Protein wird gereinigt.

4. Verfahren nach einem der Ansprüche 1 bis 3, **dadurch gekennzeichnet, dass** es sich bei dem Vektor um einen Phagemidvektor handelt, der die für OmpA-Signalpeptid kodierende DNA und die für gpIII kodierende DNA umfasst.

5. Verfahren nach einem der Ansprüche 1 bis 4, **dadurch gekennzeichnet, dass** es sich bei dem Vektor um das pComb3HSS-Phagemid handelt.

6. Verfahren nach einem der Ansprüche 1 bis 5, **dadurch gekennzeichnet, dass** die DNA-Sequenz von OmpA aus der folgenden Sequenz besteht:

7. Verfahren nach einem der Ansprüche 1 bis 6, **dadurch gekennzeichnet, dass** der für das Signalpeptid des äußeren Membranproteins A (OmpA) kodierenden DNA, die strangabwärts mit einer für ein kurzes Peptid, das durch die Aminosäuresequenz SEGNSD (SEQ ID NO:3) charakterisiert ist, kodierenden DNA fusioniert ist, die ihrerseits strangabwärts mit einer für die Kringle 2-plus-Serin-Protease-Domäne von humanem Gewebe-Plasminogenaktivator oder einer funktionellen Variante davon kodierenden DNA fusioniert ist, ein lac-promotor und/oder eine ribosomale Bindungsstelle vorausgeht.

## Revendications

1. Procédé pour la production d'un domaine kringle 2 plus sérine protéase (K2S) actif et correctement replié d'activateur de plasminogène tissulaire humain ou d'un variant fonctionnel de celui-ci dans le surnageant de cellules procaryotes, **caractérisé en ce que** la cellule procaryote contient et exprime un vecteur comprenant un ADN codant le peptide signal de protéine A de membrane externe (OmpA), qui est fusionné en aval à un ADN codant un court peptide **caractérisé par** la séquence d'aminoacides SEGNSD (SEQ ID NO : 3), qui est lui-même fusionné en aval à un ADN codant le domaine kringle 2 plus sérine protéase d'activateur de plasminogène tissulaire humain ou un variant fonctionnel de celui-ci.

2. Procédé selon la revendication 1 **caractérisé en ce que** la cellule procaryote est E. coli.

3. Procédé selon la revendication 1 ou 2 **caractérisé en ce que** les étapes suivantes sont accomplies :
a) l'ADN codant le domaine kringle 2 plus sérine protéase est amplifié par PCR ;
b) le produit de PCR est purifié ;
c) ledit produit de PCR est inséré dans un vecteur comprenant l'ADN codant le peptide signal de OmpA et l'ADN codant gpIII de telle manière que ledit produit de PCR est lié de manière fonctionnelle en amont à l'ADN codant la séquence signal de OmpA et lié en aval à l'ADN codant gpIII dudit vecteur ;
d) qu'un codon d'arrêt est inséré entre ledit domaine kringle 2 plus sérine protéase et gpIII ;
e) ledit vecteur est exprime par la cellule procaryote;
f) la protéine de domaine kringle 2 plus sérine protéase est purifiée.

4. Procédé selon l'une des revendications 1 à 3 **caractérisé en ce que** le vecteur est un vecteur phagémide comprenant l'ADN codant le peptide signal de OmpA et l'ADN codant gpIII.

5. Procédé selon l'une des revendications 1 à 4 **caractérisé en ce que** le vecteur est le phagémide pComb3HSS.

6. Procédé selon l'une des revendications 1 à 5 **caractérisé en ce que** la séquence d'ADN de OmpA consiste en la séquence suivante :

7. Procédé selon l'une des revendications 1 à 6 **caractérisé en ce que** l'ADN codant le peptide signal de protéine A de membrane externe (OmpA), qui est lui-même fusionné en aval à un ADN codant un court peptide **caractérisé par** la séquence d'aminoacides SEGNSD (SEQ ID NO : 3), qui est lui-même fusionné en aval à un AD codant le domaine kringle 2 plus sérine protéase d'activateur de plasminogène tissulaire humain ou un variant fonctionnel de celui-ci est précédé par un promoteur lac et/ou un site de fixation ribosomique.
